(19) Europäisches Patentamt
European Patent Office
Office européen des brevets

(11) **EP 4 324 386 B1**

(12) **EUROPEAN PATENT SPECIFICATION**

(45) Date of publication and mention
of the grant of the patent:
**25.02.2026 Bulletin 2026/09**

(21) Application number: **22806347.5**

(22) Date of filing: **31.03.2022**

(51) International Patent Classification (IPC):
***A61B 5/053*** *(2021.01)*

(52) Cooperative Patent Classification (CPC):
**A61B 5/0537; A61B 5/00; A61B 5/053;
A61B 5/4872; A61B 5/6898;** A61B 5/1072

(86) International application number:
**PCT/CN2022/084397**

(87) International publication number:
**WO 2022/237377 (17.11.2022 Gazette 2022/46)**

(54) **METHOD AND SYSTEM FOR MEASURING PHYSIOLOGICAL PARAMETERS, AND
ELECTRONIC DEVICE**

VERFAHREN UND SYSTEM ZUR MESSUNG PHYSIOLOGISCHER PARAMETER UND
ELEKTRONISCHE VORRICHTUNG

PROCÉDÉ ET SYSTÈME DE MESURE DE PARAMÈTRES PHYSIOLOGIQUES, ET DISPOSITIF
ÉLECTRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2021 CN 202110531351**

(43) Date of publication of application:
**21.02.2024 Bulletin 2024/08**

(73) Proprietor: **Huawei Technologies Co., Ltd.
Shenzhen, Guangdong 518129 (CN)**

(72) Inventors:
• **LIU, Chang**
**Shenzhen, Guangdong 518129 (CN)**
• **YAN, Jiabing**
**Shenzhen, Guangdong 518129 (CN)**
• **ZHAO, Shuai**
**Shenzhen, Guangdong 518129 (CN)**
• **REN, Huichao**
**Shenzhen, Guangdong 518129 (CN)**

(74) Representative: **Maiwald GmbH
Engineering
Elisenhof
Elisenstrasse 3
80335 München (DE)**

(56) References cited:
CN-A- 1 394 127        CN-A- 106 666 902
CN-A- 110 800 067      CN-A- 111 887 847
CN-U- 205 597 918      JP-A- 2002 085 365
JP-A- 2013 116 258     JP-B2- 4 723 078
US-A1- 2002 123 695    US-A1- 2009 018 464
US-A1- 2010 106 045

• ORKIN SARAH ET AL: "Body composition
measured by bioelectrical impedance analysis is
a viable alternative to magnetic resonance
imaging in children with nonalcoholic fatty liver
disease", vol. 46, no. 2, 30 April 2021
(2021-04-30), US, pages 378 - 384, XP093194195,
ISSN: 0148-6071, Retrieved from the Internet
<URL:https://onlinelibrary.wiley.com/doi/
full-xml/10.1002/jpen.2113> DOI: 10.1002/
jpen.2113

EP 4 324 386 B1

## Description

This application claims priority to Chinese Patent Application No. 202110531351.5, filed with the China National Intellectual Property Administration on May 14, 2021 and entitled "METHOD AND SYSTEM FOR MEASURING PHYSIOLOGICAL PARAMETERS AND ELECTRONIC DEVICE".

## TECHNICAL FIELD

This application relates to the field of terminal technologies, and in particular, to a method and system for measuring physiological parameters and an electronic device.

## BACKGROUND

With continuous improvement of living standards, fatty liver (Fatty liver) has gradually become the largest liver disease in humans, with a gradually increasing prevalence. The fatty liver is mainly caused by liver lesions due to excessive fat accumulation in hepatocytes. Generally, fatty liver is diagnosed if fat in the liver exceeds 5% of the liver by weight or fat degeneration occurs on more than 50% of liver cells in histology. Therefore, liver fat is an important feature in fatty liver detection. Exemplary electronic devices for measuring physiological parameters are described in e.g., JP 4 723078 B2, US 2002/123695 A1, US 2009/018464 A1, US 2010/106045 A1, and Orkin Sarah et al: "Body composition measured by bioelectrical impedance analysis is a viable alternative to magnetic resonance imaging in children with nonalcoholic fatty liver disease", JPEN - Journal of Parenteral and Enteral Nutrition, vol. 46, no. 2, 30 April 2021, pages 378-384.

In conventional technology, in addition to an abdominal B ultrasound fatty liver screening method with complex operations in medicine, a body mass index (body mass index, BMI) and a body fat ratio (body fat ratio, BFR) may be usually used for fatty liver screening. During fatty liver screening, a liver fat level may be evaluated based on a specified BMI threshold and a specified body fat ratio threshold. However, it is found by medical research that two people with the same body fat ratio may have different fatty liver risk levels. In addition, the "BMI" cannot accurately reflect obesity of a person. In other words, it is difficult to accurately evaluate the liver fat level simply through the BMI and the body fat ratio. Therefore, how to enable a user to conveniently and accurately obtain a liver fat level of the user is a technical problem that urgently needs to be resolved currently.

## SUMMARY

This application provides a method and system for measuring physiological parameters and an electronic device, so that a user can conveniently and accurately measure physiological parameters of the user. The invention is set out in the appended set of claims.

According to a first aspect, this application provides a method for measuring physiological parameters. The method includes: determining a first body parameter and a second body parameter of a to-be-measured person, where the first body parameter and the second body parameter are measured by a first electronic device, and the second body parameter is measured based on at least eight electrodes of the first electronic device; determining a third body parameter in response to an input of the to-be-measured person; determining a fourth body parameter based on the first body parameter and the third body parameter; determining a first body shape of the to-be-measured person based on the second body parameter; determining a first physiological parameter based on at least the first body shape and the fourth body parameter; and displaying the first physiological parameter. The first body parameter is a body weight, the second body parameter is a body impedance or raw data (for example, a voltage, a current, or the like) required for calculating a body impedance, the third body parameter is a body height, the fourth body parameter is a body mass index BMI, and the first physiological parameter is a liver fat level. In an example, the body impedance may include an arms-related impedance, a legs-related impedance, and a torso-related impedance. For example, the arms-related impedance may include a two-arm impedance, a left-arm impedance, a right-arm impedance, and the like. The legs-related impedance may include a two-leg impedance, a left-leg impedance, a right-leg impedance, and the like. The torso-related impedance may include a torso impedance, another impedance including a torso impedance, and the like.

Therefore, a body shape of the to-be-measured person is determined by using the first body parameter measured by the first electronic device, and the physiological parameters of the to-be-measured person are determined by combining the body shape, the first body parameter measured by the first electronic device, and the third body parameter determined based on data entered by a user, so that the physiological parameters of the to-be-measured person are accurately measured.

In a possible implementation, the determining a first body shape of the to-be-measured person based on the second body parameter specifically includes: determining a fifth body parameter based on the second body parameter; if the fifth body parameter belongs to a first interval, determining the first body shape of the to-be-measured person based on the fourth body parameter; and if the fifth body parameter belongs to a second interval, determining the first body shape of the to-be-measured person based on a sixth body parameter, where the sixth body parameter is obtained based on the second body parameter. The fifth body parameter is a waist-to-hip ratio, and the sixth body parameter is muscle mass, fat mass, or the like of the arms of the to-be-measured person. Therefore, after the fifth body parameter is determined based on the second body parameter, an appropriate body parameter may be selected based on an interval range to which the fifth body parameter belongs to determine the body shape of the to-be-measured person, to improve accuracy of determining the body shape.

In a possible implementation, the determining a fifth body parameter based on the second body parameter specifically includes: determining a seventh body parameter based on the second body parameter, and determining the fifth body parameter based on the seventh body parameter. The seventh body parameter is fat mass of each section of the body, for example, left-arm fat mass, right-arm fat mass, torso fat mass, left-leg fat mass, right-leg fat mass, or the like. Therefore, the fifth body parameter is obtained by using the second body parameter.

In a possible implementation, the determining a first physiological parameter based on at least the first body shape and the fourth body parameter specifically includes: querying a predetermined correspondence between a body shape and a detection model based on the first body shape, to determine a detection model corresponding to the first body shape, where different body shapes correspond to different detection models in the correspondence; and inputting at least the fourth body parameter into the detection model corresponding to the first body shape, to determine the first physiological parameter. Therefore, a detection model that matches the body shape of the to-be-measured person is determined based on the body shape of the to-be-measured person, and the physiological parameters of the to-be-measured person are detected by using the detection model, so that physiological parameters are detected for users of different body shapes by using different detection models, to improve accuracy of the physiological parameters.

In a possible implementation, after the displaying the first physiological parameter, the method further includes: determining a first photo and a second photo of the to-be-measured person in response to a first operation of the to-be-measured person, where the first photo is taken when the to-be-measured person performs a first preset action, and the second photo is taken when the to-be-measured person performs a second preset action; determining an eighth body parameter of the to-be-measured person based on the first photo and the second photo; redetermining the first physiological parameter based on at least the first body shape, the fourth body parameter, and the eighth body parameter; and displaying the first physiological parameter. The eighth body parameter is a waist circumference. Therefore, body parameters that are strongly correlated to the physiological parameters of the to-be-measured person are determined by using a photo of the to-be-measured person, and the physiological parameters of the to-be-measured person are accurately measured by using the body parameters, to improve detection precision of the physiological parameters.

In a possible implementation, the determining a second physiological parameter based on at least the first body shape, the fourth body parameter, and the eighth body parameter specifically includes: querying a predetermined correspondence between a body shape and a detection model based on the first body shape, to determine a detection model corresponding to the first body shape, where different body shapes correspond to different detection models in the second correspondence; and inputting at least the fourth body parameter and the eighth body parameter into the detection model corresponding to the first body shape, to redetermine the first physiological parameter. Therefore, detection accuracy of the physiological parameters is improved.

In a possible implementation, before the inputting at least the fourth body parameter and the eighth body parameter into the detection model corresponding to the first body shape, the method further includes: determining a ninth body parameter of the to-be-measured person based on the first photo and the second photo; and determining a second body shape of the to-be-measured person based on the eighth body parameter and the ninth body parameter. The ninth body parameter is a hip circumference. Because the body shape of the to-be-measured person can be accurately presented in a photo, the body shape of the to-be-measured person can be accurately determined by using a photo of the to-be-measured person, to improve detection accuracy of the body shape.

In a possible implementation, the method further includes: if the second body shape is inconsistent with the first body shape, querying the correspondence based on the second body shape, to determine a detection model corresponding to the second body shape, and selecting the detection model corresponding to the second body shape to redetermine the first physiological parameter. Therefore, the body shape of the to-be-measured person detected by using a photo is used as a reference, and the physiological parameters of the to-be-measured person are detected, to improve detection accuracy of the physiological parameters.

In a possible implementation, the determining a first photo and a second photo of the to-be-measured person specifically includes: determining an action difference degree between an action of the to-be-measured person in a currently obtained target photo and a target preset action, and determining that the action difference degree falls within a preset range, where the target photo is the first photo, and the target preset action is the first preset action; or the target photo is the second photo, and the target preset action is the second preset action. Therefore, when the to-be-measured person performs a preset action, an acquired photo is used for detection, to improve detection accuracy of the physiological parameters.

In a possible implementation, the method further includes: displaying a body shape of the to-be-measured person. In this way, the to-be-measured person can view the body shape of the to-be-measured person.

In a possible implementation, the second body parameter is measured by the first electronic device by controlling the at least eight electrodes to generate at least two electrical signals with different frequencies. In this way, the second body parameter is measured by using electrical signals with different frequencies, to improve precision of subsequent detection.

In a possible implementation, the method is performed by the first electronic device. In this way, the physiological parameters of the to-be-measured person are detected by using one device.

In a possible implementation, the method is performed by a second electronic device. The second electronic device is communicatively connected to the first electronic device; and the determining a first body parameter and a second body parameter of a to-be-measured person specifically includes: receiving, by the second electronic device, the first body parameter and the second body parameter that are sent by the first electronic device. Therefore, the physiological parameters of the to-be-measured person are detected by using a plurality of electronic devices.

According to a second aspect, this application provides a system for measuring physiological parameters. The system includes a first electronic device and a second electronic device, the first electronic device is communicatively connected to the second electronic device, and the first electronic device has at least eight electrodes.

The first electronic device is configured to: determine a first body parameter and a second body parameter of a to-be-measured person, and send the first body parameter and the second body parameter to the second electronic device, where the second body parameter is measured based on the at least eight electrodes.

The second electronic device is configured to determine a third body parameter in response to an input of the to-be-measured person. The second electronic device is further configured to: in response to receiving the first body parameter and the second body parameter, determine a fourth body parameter based on the first body parameter and the third body parameter; the second electronic device is further configured to determine a first body shape of the to-be-measured person based on the second body parameter; and the second electronic device is further configured to: determine a first physiological parameter based on at least the first body shape and the fourth body parameter, and display the first physiological parameter.

In a possible implementation, the second electronic device is further configured to: determine a fifth body parameter based on the second body parameter; if the fifth body parameter belongs to a first interval, determine the first body shape of the to-be-measured person based on the fourth body parameter; and if the fifth body parameter belongs to a second interval, determine the first body shape of the to-be-measured person based on a sixth body parameter, where the sixth body parameter is obtained based on the second body parameter.

In a possible implementation, the second electronic device is further configured to: determine a seventh body parameter based on the second body parameter, and determine the fifth body parameter based on the seventh body parameter.

In a possible implementation, the second electronic device is further configured to: query a predetermined correspondence between a body shape and a detection model based on the first body shape, to determine a detection model corresponding to the first body shape, where different body shapes correspond to different detection models in the correspondence; and input at least the fourth body parameter into the detection model corresponding to the first body shape, to determine the first physiological parameter.

In a possible implementation, after displaying the first physiological parameter, the second electronic device is further configured to: determine a first photo and a second photo of the to-be-measured person in response to a first operation of the to-be-measured person, where the first photo is taken when the to-be-measured person performs a first preset action, and the second photo is taken when the to-be-measured person performs a second preset action; determine an eighth body parameter of the to-be-measured person based on the first photo and the second photo; redetermine the first physiological parameter based on at least the first body shape, the fourth body parameter, and the eighth body parameter; and display the first physiological parameter.

In a possible implementation, the second electronic device is further configured to: query a predetermined correspondence between a body shape and a detection model based on the first body shape, to determine a detection model corresponding to the first body shape, where different body shapes correspond to different detection models in the second correspondence; and input at least the fourth body parameter and the eighth body parameter into the detection model corresponding to the first body shape, to determine the first physiological parameter.

In a possible implementation, before inputting the fourth body parameter and the eighth body parameter into the detection model corresponding to the first body shape, the second electronic device is further configured to: determine a ninth body parameter of the to-be-measured person based on the first photo and the second photo; and determine a second body shape of the to-be-measured person based on the eighth body parameter and the ninth body parameter.

In a possible implementation, the second electronic device is further configured to: if the second body shape is inconsistent with the first body shape, query the correspondence based on the second body shape, to determine a detection model corresponding to the second body shape, and select the detection model to determine the first physiological parameter.

In a possible implementation, the second electronic device is further configured to: determine an action difference degree between an action of the to-be-measured person in a currently obtained target photo and a target preset action, and determine that the action difference degree falls within a preset range, where the target photo is the first photo, and the target preset action is the first preset action; or the target photo is the second photo, and the target preset action is the second preset action.

In a possible implementation, the second electronic device is further configured to display a body shape of the to-be-measured person.

In a possible implementation, the first electronic device is further configured to: control the at least eight electrodes to generate at least two electrical signals with different frequencies; and separately determine body parameters measured based on the electrical signals with the frequencies, to obtain the second body parameter.

According to a third aspect, this application provides an apparatus for measuring physiological parameters. The apparatus includes:

a determining module, configured to determine a first body parameter and a second body parameter of a to-be-measured person, where the first body parameter and the second body parameter are measured by a first electronic device, and the second body parameter is measured based on at least eight electrodes of the first electronic device;
an input module, configured to determine a third body parameter in response to an input of the to-be-measured person;
a processing module, configured to determine a fourth body parameter based on the first body parameter and the third body parameter,
the processing module being further configured to determine a first body shape of the to-be-measured person based on the second body parameter;
the processing module being further configured to determine a first physiological parameter based on at least the first body shape and the fourth body parameter; and
a display module, configured to display the first physiological parameter.

In a possible implementation, the processing module is further configured to determine a fifth body parameter based on the second body parameter, where if the fifth body parameter belongs to a first interval, the processing module determines the first body shape of the to-be-measured person based on the fourth body parameter. If the fifth body parameter belongs to a second interval, the processing module determines the first body shape of the to-be-measured person based on a sixth body parameter, where the sixth body parameter is obtained based on the second body parameter.

In a possible implementation, the processing module is further configured to: determine a seventh body parameter based on the second body parameter, and determine the fifth body parameter based on the seventh body parameter.

In a possible implementation, the processing module is further configured to: query a predetermined correspondence between a body shape and a detection model based on the first body shape, to determine a detection model corresponding to the first body shape, where different body shapes correspond to different detection models in the correspondence; and input at least the fourth body parameter into the detection model corresponding to the first body shape, to determine the first physiological parameter.

In a possible implementation, after displaying the first physiological parameter on the display module, the processing module is further configured to: determine a first photo and a second photo of the to-be-measured person in response to a first operation of the to-be-measured person, where the first photo is taken when the to-be-measured person performs a first preset action, and the second photo is taken when the to-be-measured person performs a second preset action; determine an eighth body parameter of the to-be-measured person based on the first photo and the second photo; redetermine the first physiological parameter based on at least the first body shape, the fourth body parameter, and the eighth body parameter; and
the display module is further configured to display the first physiological parameter.

6. The method according to claim 5, where the processing module is further configured to: query a predetermined correspondence between a body shape and a detection model based on the first body shape, to determine a detection model corresponding to the first body shape, where different body shapes correspond to different detection models in the second correspondence; and input at least the fourth body parameter and the eighth body parameter into the detection model corresponding to the first body shape, to redetermine the first physiological parameter.

In a possible implementation, the processing module is further configured to: determine a ninth body parameter of the to-be-measured person based on the first photo and the second photo; and determine a second body shape of the to-be-measured person based on the eighth body parameter and the ninth body parameter.

In a possible implementation, when the second body shape is inconsistent with the first body shape, the processing module queries the correspondence based on the second body shape, to determine a detection model corresponding to the second body shape, and selects the detection model corresponding to the second body shape to redetermine the first physiological parameter.

In a possible implementation, the processing module is further configured to: determine an action difference degree between an action of the to-be-measured person in a currently obtained target photo and a target preset action, and determine that the action difference degree falls within a preset range.

The target photo is the first photo, and the target preset action is the first preset action; or the target photo is the second photo, and the target preset action is the second preset action.

In a possible implementation, the display module is further configured to display a body shape of the to-be-measured person.

In a possible implementation, the second body parameter is measured by the first electronic device by controlling the at least eight electrodes to generate at least two electrical signals with different frequencies.

In a possible implementation, the apparatus is deployed on the first electronic device.

In a possible implementation, the apparatus is deployed on a second electronic device, and the second electronic device is communicatively connected to the first electronic device. The second electronic device may receive, from the first electronic device, the first body parameter and the second body parameter that are sent by the first electronic device.

According to a fourth aspect, this application provides an electronic device, including: at least one memory, configured to store a program; and at least one processor, configured to execute the program stored in the memory, where when the program stored in the memory is executed, the processor is configured to perform the method provided in the first aspect.

According to a fifth aspect, this application provides a computer storage medium. The computer storage medium stores instructions, and when the instructions are run on a computer, the computer is enabled to perform the method provided in the first aspect.

According to a sixth aspect, this application provides a computer program product including instructions. When the instructions are run on a computer, the computer is enabled to perform the method provided in the first aspect.

## BRIEF DESCRIPTION OF DRAWINGS

FIG. 1a is a schematic diagram of a system architecture of a system for detecting a liver fat level according to an embodiment of this application;

FIG. 1b is a schematic diagram of a system architecture of another system for detecting a liver fat level according to an embodiment of this application;

FIG. 2 is a schematic diagram of a hardware structure of a body fat scale according to an embodiment of this application;

FIG. 3 is a schematic diagram of a hardware structure of an electronic device according to an embodiment of this application;

FIG. 4 is a schematic diagram of a scenario in which a to-be-measured person uses a body fat scale according to an embodiment of this application;

FIG. 5 is a schematic diagram of resistors corresponding to sections of the body of a to-be-measured person according to an embodiment of this application;

FIG. 6 is a schematic diagram of a display interface of an electronic device according to an embodiment of this application;

FIG. 7 is a schematic diagram of a display interface of an electronic device according to an embodiment of this application;

FIG. 8 is a schematic diagram of a display interface of an electronic device according to an embodiment of this application;

FIG. 9a is a schematic diagram of a display interface of an electronic device according to an embodiment of this application;

FIG. 9b is a schematic diagram of a display interface of an electronic device according to an embodiment of this application;

FIG. 10a is a schematic diagram of a display interface of a mobile phone according to an embodiment of this application;

FIG. 10b is a schematic diagram of a display interface of a mobile phone according to an embodiment of this application;

FIG. 10c is a schematic diagram of a display interface of a mobile phone according to an embodiment of this application;

FIG. 10d is a schematic diagram of a display interface of a mobile phone according to an embodiment of this application;

FIG. 10e is a schematic diagram of a display interface of a mobile phone according to an embodiment of this application;

FIG. 10f is a schematic diagram of a display interface of a mobile phone according to an embodiment of this application;

FIG. 11a is a schematic diagram of a display interface of a large screen according to an embodiment of this application;

FIG. 11b is a schematic diagram of a display interface of a large screen according to an embodiment of this application;

FIG. 11c is a schematic diagram of a display interface of a large screen according to an embodiment of this application;

FIG. 11d is a schematic diagram of a display interface of a large screen according to an embodiment of this application;

FIG. 11e is a schematic diagram of a display interface of a large screen according to an embodiment of this application;

FIG. 11f is a schematic diagram of a display interface of a large screen according to an embodiment of this application;

FIG. 11g is a schematic diagram of a display interface of a large screen according to an embodiment of this application;

FIG. 12 is a schematic flowchart of a method for measuring physiological parameters according to an embodiment of this application;

FIG. 13 is a schematic diagram of steps of precisely measuring a first physiological parameter of a to-be-measured person according to an embodiment of this application; and

FIG. 14 is a schematic diagram of an architecture of a system for measuring physiological parameters according to an embodiment of this application.

## DESCRIPTION OF EMBODIMENTS

To make objectives, technical solutions, and advantages of embodiments of this application clearer, the following describes the technical solutions in embodiments of this application with reference to accompanying drawings.

In descriptions of embodiments of this application, words such as "example", "for example", or "in an example" are used to indicate giving an example, an illustration, or a description. Any embodiment or design scheme described as an "example", "for example", or "for example" in embodiments of this application should not be explained as being more preferred or having more advantages than another embodiment or design scheme. Exactly, use of the words such as "example", "for example", "in an example" is intended to present a relative concept in a specific manner.

In the descriptions of embodiments of this application, a term "and/or" describes only an association relationship for describing associated objects and indicates that three relationships may exist. For example, A and/or B may indicate the following three cases: Only A exists, both A and B exist, and only B exists. In addition, unless otherwise specified, a term "plurality" means two or more. For example, a plurality of systems are two or more systems, and a plurality of electronic devices are two or more electronic devices.

Moreover, terms "first" and "second" are merely intended for description, and shall not be understood as an indication or implication of relative importance or implicit indication of an indicated technical feature. Therefore, a feature limited by "first" or "second" may explicitly or implicitly include one or more features. The terms "include", "comprise", "have", and their variants all mean "include but are not limited to", unless otherwise specifically emphasized in another manner.

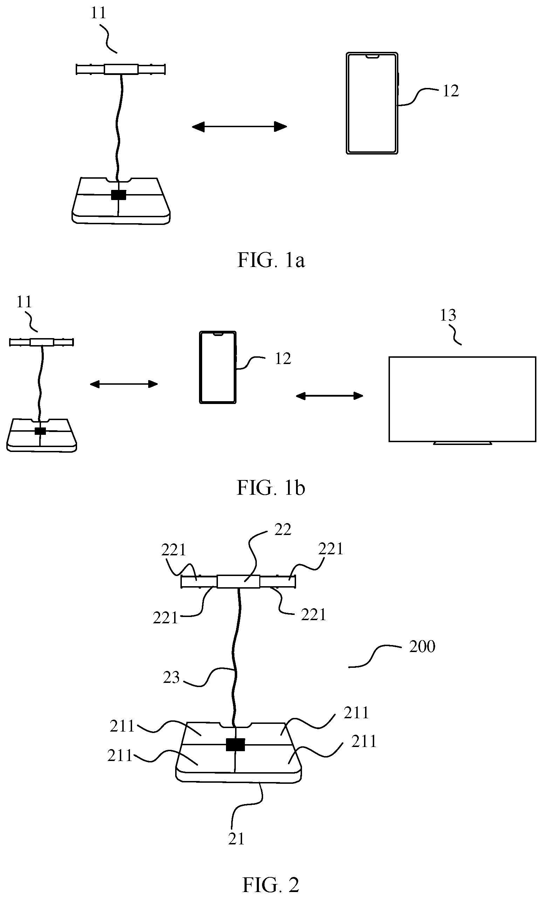

FIG. 1a is a schematic diagram of a system architecture of a system for detecting a liver fat level according to an embodiment of this application. As shown in FIG. 1a, the system includes a body fat scale 11 and a mobile phone 12. A connection may be established between the body fat scale 11 and the mobile phone 12 via Bluetooth.

The body fat scale 11 may detect a body weight and a body impedance of a to-be-measured person. The body impedance may include a two-arm impedance, a two-leg impedance, and a torso impedance. In an example, after obtaining the body weight and the body impedance of the to-be-measured person, the body fat scale 11 may send the body weight and the body impedance to the mobile phone 12 via Bluetooth. In addition, if no connection is established between the body fat scale 11 and the mobile phone 12 when the body fat scale 11 measures the body weight and the body impedance, after the connection is subsequently established between the body fat scale 11 and the mobile phone 12, data may be synchronized between the body fat scale 11 and the mobile phone 12, in other words, the body fat scale 11 sends the data measured by the body fat scale to the mobile phone 12. In addition, the body fat scale 11 may further send, to the mobile phone 12, basic data that is detected by the body fat scale and that is required for calculating the body impedance, and then the mobile phone 12 may obtain the body impedance through calculation based on the basic data.

The mobile phone 12 may determine a body parameter of the to-be-measured person based on the body weight and the body impedance that are detected by the body fat scale 11. For example, the body parameter may include a body mass index BMI, a body fat ratio, a visceral fat level, fat mass of each section of the body, a waist-to-hip ratio, a body shape, or the like. For example, the body shape may include an apple type, a pear type, a red hot chili type, an hourglass type, an inverted triangle type, or the like. Next, the mobile phone 12 may further determine, based on the body shape of the to-be-measured person, a level determination model corresponding to the body shape. Then, the mobile phone 12 may input the body parameter of the to-be-measured person into the level determination model, to obtain a liver fat level of the to-be-measured person. Finally, the mobile phone 12 may present the determined liver fat level to the to-be-measured person.

In this solution, in a process of determining the liver fat level, for users of different body shapes, different level determination models may be used to calculate liver fat levels of the users, so that different processing manners are used for different people, evaluation accuracy of a liver fat level is improved, and a case in which one liver fat level evaluation manner is used for all people is avoided.

It may be understood that, in this solution, some or all functions implemented by the mobile phone 12 may alternatively be implemented by the body fat scale 11 or an electronic device other than the body fat scale 11. This is not limited herein. For example, the body fat ratio may be obtained through calculation by the body fat scale 11. In other words, the body fat scale 11 may transmit measured data to the mobile phone 12, and may further obtain some data through calculation and transmit the calculated data to the mobile phone 12. For example, as shown in FIG. 1b, the system may further include a smart screen 13. For example, a connection may be established between the body fat scale 11 and the mobile phone 12 via Bluetooth, or a connection may be established between the mobile phone 12 and the smart screen 13 through a wireless network. The smart screen 13 may implement some functions implemented by the mobile phone 12, for example, present, to the to-be-measured person, the liver fat level determined by the mobile phone 12. For example, after determining the liver fat level of the to-be-measured person based on the body impedance and the body weight of the to-be-measured person that are sent by the body fat scale 11, the mobile phone 12 may cast the determined liver fat level to the smart screen 13 through screen casting, and then the smart screen 13 may present the liver fat level to the to-be-measured person.

It may be understood that, in this solution, the body fat scale 11 may be replaced with another electronic device, and the electronic device may implement a function implemented by the body fat scale 11 in this solution. This is not limited herein. For example, the electronic device in place of the body fat scale 11 may have at least a function of detecting a body impedance and a body weight of a to-be-measured person. The mobile phone 12 may be replaced with another electronic device, and the electronic device may implement the function implemented by the mobile phone 12 in this solution. This is not limited herein. For example, the electronic device in place of the mobile phone 12 may be a tablet computer, a wearable device, a smart television, a smart screen, or the like. The smart screen 13 may be replaced with another electronic device, and the electronic device may implement a function implemented by the smart screen 13 in this solution. This is not limited herein. For example, the electronic device in place of the smart screen 13 may be a tablet computer, a wearable device, a smart television, or the like.

In addition, in this solution, a connection may be established between the body fat scale 11 and the mobile phone 12 in another connection manner, and a connection may be established between the mobile phone 12 and the smart screen 13 in another connection manner. This is not limited herein. For example, a connection may be established between the body fat scale 11 and the mobile phone 12 by using a short-range wireless connection technology or a long-range wireless connection technology. The short-range wireless connection technology may include ZigBee (ZigBee), and the like. The long-range wireless connection technology may include wireless fidelity (wireless fidelity, Wi-Fi), cellular mobile communication (cellular mobile communication), and the like. A connection may be established between the mobile phone 12 and the smart screen 13 by using the short-range wireless connection technology or the long-range wireless connection technology. The short-range wireless connection technology may include ZigBee (ZigBee), and the like. The long-range wireless connection technology may include wireless fidelity (wireless fidelity, Wi-Fi), cellular mobile communication (cellular mobile communication), and the like.

Next, a schematic diagram of a hardware structure of a body fat scale provided in this solution is described. For example, the body fat scale may be the body fat scale 11 shown in FIG. 1a or FIG. 1b.

FIG. 2 is a schematic diagram of a hardware structure of a body fat scale according to an embodiment of this application. As shown in FIG. 2, a body fat scale 200 may include a scale body 21 and a handle 22. The scale body 21 and the handle 22 may be connected by a cable 23. At least four electrodes 211 may be disposed on the scale body 21. At least four electrodes 221 may be disposed on the handle 22. When a user uses the body fat scale 200, at least two electrodes on the scale body 21 are in contact with the left foot of the user, and at least two electrodes are in contact with the right foot of the user; and at least two electrodes on the handle 22 are in contact with the left hand of the user, and at least two electrodes are in contact with the right hand of the user.

It may be understood that a pressure sensor (not shown in the figure) may be further disposed in the scale body 21. A body weight of the user may be detected by using the pressure sensor. In addition, a display 222 may be further disposed on the handle 22, and the display 222 may display the body weight, a body fat ratio, and the like of the user.

In this solution, a processor (not shown in the figure) and a communication module (not shown in the figure) may be further disposed in the body fat scale 200. The processor in the body fat scale 200 may determine the body weight of the user based on a signal detected by the pressure sensor in the scale body 21, determine a body impedance of the user based on signals detected by the electrodes on the scale body 21 and the handle 22, determine the body fat ratio of the user based on the determined body impedance, and the like.

The communication module in the body fat scale 200 may provide short-range communication or long-range communication for the body fat scale 200, to implement information exchange between the body fat scale 200 and another electronic device. For example, the communication module in the body fat scale 200 may use Bluetooth (Bluetooth), ZigBee (ZigBee), wireless fidelity (wireless fidelity, Wi-Fi), cellular mobile communication (cellular mobile communication), or the like.

It may be understood that the structure shown in FIG. 2 in this solution does not constitute a specific limitation on the body fat scale. In some other embodiments of this solution, the body fat scale may include more or fewer components than those shown in the figure, or some components may be combined, or some components may be split, or different component arrangements may be used. The components shown in the figure may be implemented by hardware, software, or a combination of software and hardware.

Next, a schematic diagram of a hardware structure of an electronic device provided in this solution is described. For example, the electronic device may be the mobile phone 12 shown in FIG. 1a or FIG. 1b, or may be the smart screen 13 shown in FIG. 1b.

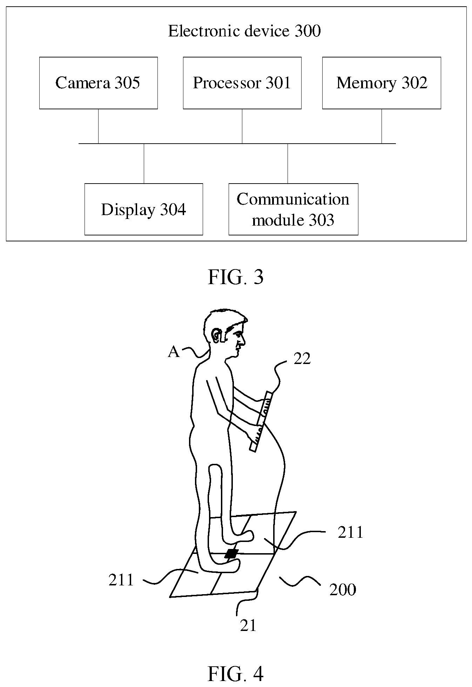

FIG. 3 is a schematic diagram of a hardware structure of an electronic device according to an embodiment of this application. As shown in FIG. 3, an electronic device 300 may include a processor 301, a memory 302, and a communication module 303. The processor 301, the memory 302, and the communication module 303 may be connected by a bus or in another manner.

In this solution, the processor 301 is a computing core and a control core of the electronic device. The processor 301 may include one or more processing units. For example, the processor 301 may include one or more of an application processor (application processor, AP), a modem (modem), a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors. In an example, the processor 301 may implement a method for detecting a liver fat level provided in this solution. For example, the processor 301 may determine a body parameter of a to-be-measured person based on a body weight and a body impedance that are detected by a body fat scale. The processor 301 may further determine, based on a body shape of the to-be-measured person, a level determination model corresponding to the body shape. The processor 301 may further input the body parameter of the to-be-measured person into the level determination model, to obtain a liver fat level of the to-be-measured person.

The memory 302 is a memory device of the electronic device, and is configured to store a program and data, for example, store a location of the electronic device, a location of another electronic device received by the electronic device, and the like. It may be understood that the memory 302 may be a high-speed RAM memory, or may be a non-volatile memory (non-volatile memory). Optionally, the memory 302 may be at least one storage apparatus located far away from the processor 301. The memory 302 may provide storage space. The storage space may store an operating system and executable program code of the electronic device, and may include, but is not limited to, a Windows system (an operating system), a Linux system (an operating system), a HarmonyOS (an operating system), and the like. This is not limited herein. For example, the memory 302 may store the level determination model used for determining a liver fat level.

The communication module 303 may provide short-range communication or long-range communication for the electronic device, to implement information exchange between the electronic device and another electronic device (for example, the body fat scale). For example, the communication module 303 may use Bluetooth (Bluetooth), ZigBee (ZigBee), wireless fidelity (wireless fidelity, Wi-Fi), cellular mobile communication (cellular mobile communication), or the like.

Optionally, the electronic device 300 may further include a display 304. For example, the display 304 may display the liver fat level, the body parameter, and the like of the to-be-measured person.

Optionally, the electronic device 300 may further include a camera 305. The camera 305 may be configured to capture a static image or a video, for example, capture an image of the to-be-measured person.

It may be understood that the structure shown in FIG. 3 in this solution does not constitute a specific limitation on the electronic device. In some other embodiments of this solution, the electronic device may include more or fewer components than those shown in the figure, or some components may be combined, or some components may be split, or different component arrangements may be used. The components shown in the figure may be implemented by hardware, software, or a combination of software and hardware.

The foregoing describes the system for detecting a liver fat level in this solution and the hardware structure of the electronic device in the system. Based on the foregoing described content, the following describes in detail a solution of detecting a liver fat level provided in this solution.

(1) Determine a body weight and a body impedance of a to-be-measured person.

In this solution, the body weight and the body impedance of the to-be-measured person may be determined by using the body fat scale 200 shown in FIG. 2. As shown in FIG. 4, a to-be-measured person A may stand on the scale body 21 of the body fat scale 200, and hold the handle 22 of the body fat scale 200. The left foot of the to-be-measured person A may be in contact with two electrodes 211 on the scale body 21, and the right foot of the to-be-measured person A may also be in contact with two electrodes 211 on the scale body 21. The left hand of the to-be-measured person A may be in contact with two electrodes (not shown in the figure) on the handle 22, and the right hand of the to-be-measured person A may also be in contact with two electrodes (not shown in the figure) on the handle 22. After the to-be-measured person A starts detection, the electrodes on the body fat scale 200 may generate electrical signals with specific frequencies to measure the body impedance of the to-be-measured person A. In this solution, the body impedance may include a left-arm impedance, a right-arm impedance, a left-leg impedance, a right-leg impedance, a torso impedance, and the like. Impedances of different areas on the body may be obtained at the same time, or may be obtained at different time. This may be specifically determined according to an actual case. This is not limited herein.

In an example, still refer to FIG. 4. When measuring the body impedance of the to-be-measured person A, the body fat scale 200 may first turn on the electrodes on the left side and the right side of the handle 22 of the body fat scale, and then measure a two-arm impedance R1 based on a power-on current and a power-on voltage. Next, the body fat scale 200 may stop turning on the electrodes on the handle 22 of the body fat scale, then turn on the electrodes on the body 21 of the body fat scale, and then measure a two-leg impedance R2 based on a power-on current and a power-on voltage. Next, the body fat scale 200 may stop turning on the electrodes on the left side and the right side of the body 21 of the body fat scale, turn on the electrodes on the left side of the handle 22 of the body fat scale and the electrodes on the right side of the body 21 of the body fat scale, and then measure a left oblique half body impedance R3 based on a power-on current and a power-on voltage. Next, the body fat scale 200 may stop turning on the electrodes on the left side of the handle 22 of the body fat scale and the electrodes on the right side of the body 21 of the body fat scale, turn on the electrodes on the right side of the handle 22 of the body fat scale and the electrodes on the left side of the body 21 of the body fat scale, and then measure a right oblique half body impedance R4 based on a power-on current and a power-on voltage. Next, the body fat scale 200 may stop turning on the electrodes on the right side of the handle 22 of the body fat scale and the electrodes on the left side of the body 21 of the body fat scale, turn on the electrodes on the left side of the handle 22 of the body fat scale and the electrodes on the left side of the body 21 of the body fat scale, and then measure a left body impedance R5 based on a power-on current and a power-on voltage. Next, the body fat scale 200 may stop turning on the electrodes on the left side of the handle 22 of the body fat scale and the electrodes on the left side of the body 21 of the body fat scale, turn on the electrodes on the right side of the handle 22 of the body fat scale and the electrodes on the right side of the body 21 of the body fat scale, and then measure a right body impedance R6 based on a power-on current and a power-on voltage. Finally, the body fat scale 200 may determine the left-arm impedance, the right-arm impedance, the left-leg impedance, the right-leg impedance, and the torso impedance of the to-be-measured person A based on the measured two-arm impedance R1, two-leg impedance R2, left oblique half body impedance R3, right oblique half body impedance R4, left body impedance R5, and right body impedance R6. It should be noted that, in this solution, a power-on sequence of the electrodes is merely an example for description, and may be specifically determined according to an actual case. This is not limited herein.

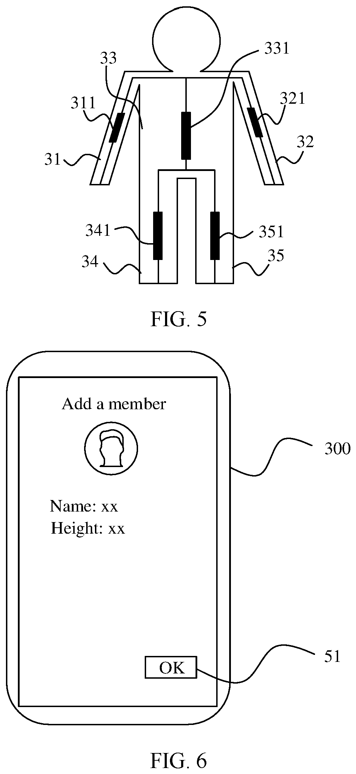

For example, as shown in FIG. 5, during measurement of the body impedance, the left arm 31 of the to-be-measured person A may be equivalent to a resistor (namely, a resistor 311), the right arm 32 of the to-be-measured person A may be equivalent to a resistor (namely, a resistor 321), the torso 33 of the to-be-measured person A may be equivalent to a resistor (namely, a resistor 331), the left leg 34 of the to-be-measured person A may be equivalent to a resistor (namely, a resistor 341), and the right leg 35 of the to-be-measured person A may be equivalent to a resistor (namely, a resistor 351). Still refer to FIG. 5. In this solution, the two-arm impedance may be a sum of resistance values of the resistor 311 and the resistor 321, in other words, the two-arm impedance may be a sum of the left-arm impedance and the right-arm impedance; the two-leg impedance may be a sum of resistance values of the resistor 341 and the resistor 351, in other words, the two-leg impedance may be a sum of the left-leg impedance and the right-leg impedance; and the torso impedance may be a resistance value of the resistor 341. Further, after the body fat scale 200 measures the two-arm impedance R1, the two-leg impedance R2, the left oblique half body impedance R3, the right oblique half body impedance R4, the left body impedance R5, and the right body impedance R6, the following data may be obtained:

$$R1 = R311 + R321;$$

$$R2 = R341 + R351;$$

$$R3 = R311 + R331 + R351;$$

$$R4 = R321 + R331 + R341;$$

$$R5 = R311 + R331 + R341;$$

and

$$R6 = R321 + R331 + R351.$$

Further, impedances of the left arm, the right arm, the left leg, the right leg, and the torso of the to-be-measured person A may be obtained through mathematical operations. $R311 = (R1 + R5 - R4)/2$, $R321 = (R1 + R6 - R3)/2$, $R331 = (R3 + R4 - R1 - R2)/2$, $R341 = (R2 + R5 - R3)/2$, and $R351 = (R2 + R6 - R4)/2$. R311 may be the left-arm impedance, R321 may be the right-arm impedance, R331 may be the torso impedance, R341 may be the left-leg impedance, and R351 may be the right-leg impedance.

In addition, when the to-be-measured person A stands on the body fat scale 200, if the body fat scale 200 is in an on state, the body fat scale 200 may measure a body weight of the to-be-measured person A by using a pressure sensor in the body fat scale.

In an example, during measurement of the body impedance of the to-be-measured person A, the electrodes on the body fat scale 200 may be controlled to generate electrical signals with a plurality of frequencies to measure the body impedance of the to-be-measured person, to obtain impedances when the electrical signals flow through an intracellular fluid in the body and impedances when the electrical signals do not flow through the intracellular fluid in the body, so that the body impedance of the to-be-measured person is measured from a plurality of dimensions, to improve detection accuracy. For example, the frequencies of the electrical signals generated by the electrodes on the body fat scale 200 may be 50 kilohertz (kHz) and 250 kilohertz (kHz). A sequence of generating the electrical signals with different frequencies may vary, and this is not limited herein. For example, a 50 kHz electrical signal is first generated, and then a 250 kHz electrical signal is generated; or a 250 kHz electrical signal is first generated, and then a 50 kHz electrical signal is generated. Because the frequency of the 50 kHz electrical signal is low, when the electrical signal with the frequency is used for measurement, it is difficult for the electrical signal to penetrate the intracellular fluid. In other words, the body impedance measured in this case is an impedance when the electrical signal does not flow through the intracellular fluid in the body. Because the frequency of the 250 kHz electrical signal is high, when the electrical signal with the frequency is used for measurement, the electrical signal may penetrate the intracellular fluid. In other words, the body impedance measured in this case is an impedance when the electrical signal flows through the intracellular fluid in the body.

(2) Determine a liver fat level of the to-be-measured person.

In this solution, after the body weight and the body impedance of the to-be-measured person are determined by using the body fat scale, the liver fat level of the to-be-measured person may be determined based on the body weight and the body impedance of the to-be-measured person.

In an example, after the body weight and the body impedance of the to-be-measured person A are determined by using the body fat scale 200 shown in FIG. 2, the body fat scale 200 may transmit the body weight and the body impedance of the to-be-measured person A to the electronic device 300 shown in FIG. 3, to determine a liver fat level of the to-be-measured person A by using the electronic device 300. In addition, the body impedance of the to-be-measured person A may alternatively be calculated by a mobile phone based on initial data measured by the body fat scale 200. The body fat scale 200 may send the two-arm impedance R1, the two-leg impedance R2, the left oblique half body impedance R3, the right oblique half body impedance R4, the left body impedance R5, and the right body impedance R5 that are measured by the body fat scale to the electronic device 300. Subsequently, the electronic device 300 obtains body impedances such as the left-arm impedance, the right-arm impedance, the left-leg impedance, the right-leg impedance, and the torso impedance through calculation.

Before determining the liver fat level of the to-be-measured person A, the electronic device 300 may first determine a basic parameter such as a body height of the to-be-measured person A. For example, an application program (for example, Huawei Health) related to a liver fat level may be installed on the electronic device 300. As shown in FIG. 6, the to-be-measured person A may input a basic parameter such as a body height into the application program related to a liver fat level. After completing the input, the to-be-measured person A may select an "OK" button in an area 51. In this way, the basic parameter such as the body height of the to-be-measured person A may be inputted into the application program related to a liver fat level.

After learning of the basic parameter such as the body height of the to-be-measured person A, the electronic device 300 may determine a body parameter such as a body mass index BMI, a body fat ratio BFR, visceral fat mass in the torso, fat mass of each section of the body, a waist-to-hip ratio, a body shape, or the like of the to-be-measured person A with reference to the body weight and the body impedance of the to-be-measured person. For example, the visceral fat mass in the torso may be understood as fat content of most of the visceral fat in the torso, or fat content of all the visceral fat in the torso.

In this solution, the body mass index is $\mathrm{BMI} = W/H^2$. W is the body weight, and H is the body height.

The body fat ratio may be calculated by using the following formula. The formula (referred to as "Formula 1" below) is:

$$\mathrm{BFR} = \alpha_1 Z1_{50} + \alpha_2 Z1_{250} + \alpha_3 Z2_{50} + \alpha_4 Z2_{250} + \alpha_5 Z3_{50} + \alpha_6 Z3_{250} + \alpha_7 w_t$$
$$+ \alpha_8 H_t + \alpha_9$$

BFR is the body fat ratio. $\alpha_1,..., \alpha_9$ are preset coefficients, and may be obtained by experiment. $Z1_{50}$ is a two-leg impedance at 50 kHz. $Z1_{250}$ is a two-leg impedance at 250 kHz. $Z2_{50}$ is a two-arm impedance at 50 kHz. $Z2_{250}$ is a two-arm impedance at 250 kHz. $Z3_{50}$ is a torso impedance at 50 kHz. $Z3_{250}$ is a torso impedance at 250 kHz. wt is the body weight. $H_t$ is the body height. In an example, after the body fat scale measures the body fat ratio, the body fat scale may send the body fat ratio to the electronic device 300, so that the electronic device 300 can directly obtain the body fat ratio. It may be understood that $Z_{50}$ and $Z_{250}$ in Formula 1 may be replaced with impedances at other frequencies. This is not limited herein.

The visceral fat mass in the torso may be calculated by the following formula. The formula (referred to as "Formula 2" below) is:

$$X = \beta_1 Z1_{50} + \beta_2 Z1_{250} + \beta_3 Z2_{50} + \beta_4 Z2_{250} + \beta_5 Z3_{50} + \beta_6 Z3_{250} + \beta_7 W_t + \beta_8 H_t, + \beta 9$$

X is the visceral fat mass in the torso. $\beta_1,..., \beta_9$ are preset coefficients, and may be obtained by experiment. $Z1_{50}$ is a two-leg impedance at 50 kHz. $Z1_{250}$ is a two-leg impedance at 250 kHz. $Z2_{50}$ is a two-arm impedance at 50 kHz. $Z2_{250}$ is a two-arm impedance at 250 kHz. $Z3_{50}$ is a torso impedance at 50 kHz. $Z3_{250}$ is a torso impedance at 250 kHz. $w_t$ is the body weight. $H_t$ is the body height. It may be understood that $Z_{50}$ and $Z_{250}$ in Formula 2 may be replaced with impedances at other frequencies. This is not limited herein.

The fat mass of each section of the body may be calculated by using the following formula. The formula (referred to as "Formula 3" below) is:

$$P = \delta_1 Z_{50} + \delta_2 Z_{250} + \delta_3 w_t + \delta_4 H_t + \delta_5$$

P is the fat mass of each section of the body, and may be left-arm fat mass, right-arm fat mass, left-leg fat mass, right-leg fat mass, or torso fat mass. $\delta_1,..., \delta_5$ are preset coefficients, and may be obtained by experiment. $Z_{50}$ is an impedance at 50 kHz. $Z_{250}$ is an impedance at 250 kHz. $w_t$ is the body weight. $H_t$ is the body height. It may be understood that, when P is the left-arm fat mass, $Z_{50}$ is a left-arm impedance at 50 kHz, and $Z_{250}$ is a left-arm impedance at 250 kHz. When P is the right-arm fat mass, $Z_{50}$ is a right-arm impedance at 50 kHz, and $Z_{250}$ is a right-arm impedance at 250 kHz. When P is the left-leg fat mass, $Z_{50}$ is a left-leg impedance at 50 kHz, and $Z_{250}$ is a left-leg impedance at 250 kHz. When P is the right-leg fat mass, $Z_{50}$ is a right-leg impedance at 50 kHz. When P is the torso fat mass, $Z_{50}$ is a torso impedance at 50 kHz, and $Z_{250}$ is a torso impedance at 250 kHz. During determining of fat mass of different sections of the body, parameters $\delta$ in Formula 3 may be partially the same, or may be completely the same, or may be completely different. This may be specifically determined according to an actual case. This is not limited herein. It may be understood that $Z_{50}$ and $Z_{250}$ in Formula 3 may be replaced with impedances at other frequencies. This is not limited herein.

The waist-to-hip ratio may be calculated by using the following formula. The formula (referred to as "Formula 4" below) is:

$$Y = \gamma_1 L1 + \gamma_2 L2 + \gamma_3 L3 + \gamma_4 L4 + \gamma_5 L5 + \gamma Y_6 L6 + \gamma_7 L7 + \gamma_8 L8 + \gamma_9 L9 + \gamma_{10} L10 + \gamma_{11}$$

Y is the waist-to-hip ratio. $\gamma_1,...,\gamma_{11}$ are preset coefficients, and may be obtained by experiment. L1 is the left-arm muscle mass. L2 is the left-arm fat mass. L3 is the right-arm muscle mass. L4 is the right-arm fat mass. L5 is the left-leg muscle mass. L6 is the left-leg fat mass. L7 is the right-leg muscle mass. L8 is the right-leg fat mass. L9 is the torso muscle mass. L10 is the torso fat mass. In an example, the "left-arm muscle mass L1", the "right-arm muscle mass L3", the "left-leg muscle mass L5", the "right-leg muscle mass L7 ", and the "torso muscle mass L9" in Formula 4 may be adaptively selected. This is not limited herein.

In this solution, the muscle mass of each section of the body may be calculated by using the following formula: The formula (referred to as "Formula 5" below) is:

$$M = \theta_1 Z_{50} + \theta_2 Z_{250} + \theta_3 W_t + \theta_4 H_t + \theta_5$$

M is the muscle mass of each section of the body, and may be the left-arm muscle mass, the right-arm muscle mass, the left-leg muscle mass, the right-leg muscle mass, or the torso fat mass. $\theta_1,..., \theta_5$ are preset coefficients, and may be obtained by experiment. $Z_{50}$ is an impedance at 50 kHz; $Z_{250}$ is an impedance at 250 kHz. $w_t$ is the body weight. $H_t$ is the body height. It may be understood that, when M is the left-leg muscle mass, $Z_{50}$ is a left-leg impedance at 50 kHz, and $Z_{250}$ is a left-leg impedance at 250 kHz. When M is the right-leg muscle mass, $Z_{50}$ is a right-leg impedance at 50 kHz, and $Z_{250}$ is a right-leg impedance at 250 kHz. When M is the left-leg muscle mass, $Z_{50}$ is a left-leg impedance at 50 kHz, and $Z_{250}$ is a left-leg impedance at 250 kHz. When M is the right-leg muscle mass, $Z_{50}$ is a right-leg impedance at 50 kHz. When M is the torso muscle mass, $Z_{50}$ is a torso impedance at 50 kHz, and $Z_{250}$ is a torso impedance at 250 kHz. During determining of muscle mass of different sections of the body, parameters $\theta$ in Formula 5 may be partially the same, or may be completely the same, or may be completely different. This may be specifically determined according to an actual case. This is not limited herein. It may be understood that $Z_{50}$ and $Z_{250}$ in Formula 5 may be replaced with impedances at other frequencies. This is not limited herein.

In this solution, after the waist-to-hip ratio is determined, the body parameter required for currently determining the body shape may be determined based on the waist-to-hip ratio, and then the body shape is determined based on the body parameter. In an example, the required body parameter may be determined based on an interval to which the waist-to-hip ratio belongs. For example, when the waist-to-hip ratio falls within a preset interval a1 (for example, a1 ∈ (0.78, 0.85)), the BMI may be selected to determine the body shape. In this case, when the BMI is less than a preset threshold b1 (for example, b1 = 21), the body shape is a red hot chili type. When the BMI is greater than or equal to the preset threshold b1, the body shape is a well-proportioned type. When the waist-to-hip ratio falls within a preset interval a2 (for example, a2 ∈ (0, 0.78]), muscle mass of the two arms may be selected to determine the body shape. In this case, when a ratio of the muscle mass of the arms to total muscle mass of the body is greater than or equal to a preset threshold b2 (for example, b2 = 0.0981), it may be determined that the body shape is an hourglass type. When the ratio of the muscle mass of the arms to the total muscle mass of the body is less than the preset threshold b2, it may be determined that the body shape is a pear type.

It may be understood that, to improve accuracy of determining a body shape, during determining of the body shape, the waist-to-hip ratio may be combined with a plurality of other body parameters. For example, the waist-to-hip ratio, the fat mass and muscle mass of each section of the body, the BMI, and the visceral fat mass in the torso may be used. The parameters used for determining the body shape may be inputted into a machine learning classification model, to obtain the body shape.

Further, after determining the body shape of the to-be-measured person A, the electronic device 300 may determine, based on the body shape, a level determination model corresponding to the body shape of the to-be-measured person A from a preset correspondence between a body shape and a level determination model used for determining a liver fat level. It may be understood that, in this solution, the level determination model used for determining a liver fat level may be obtained through training by using a Gaussian process model, a neural network model, a support vector machine, or the like. In addition, the level determination model may be a bias function model, a proportional function model, a confounding function model, or another mathematical function model. For example, the preset correspondence between a body shape and a level determination model used for determining a liver fat level may be shown in Table 1. When it is determined that the body shape is "Apple type", it may be learned from Table 1 that a level determination model that should be selected in this case is "Model 2".

**Table 1**

| Body shape | Level determination model |
|---|---|
| Pear type | Model 1 |
| Apple type | Model 2 |
| Hourglass type | Model 3 |
| Red hot chili type | Model 4 |

Further, after determining the level determination model, the electronic device 300 may input body parameters such as the BMI, the body fat ratio, the waist-to-hip ratio, and the like of the to-be-measured person A into the level determination model, and may obtain the liver fat level of the to-be-measured person A after processing by using the level determination model. It may be understood that the parameters inputted into the level determination model may include other body parameters, for example, the visceral fat mass in the torso, the fat mass of each section of the body, the body shape, and the like, to improve detection accuracy.

Next, the electronic device 300 may determine a liver risk level of the to-be-measured person A based on a correspondence between a liver fat level and a liver risk coefficient. For example, a preset correspondence between a liver fat level and a liver risk level may be shown in Table 2. When it is determined that the liver fat level is "5", it can be learned from Table 2 that the liver risk level in this case is "Suspected risk".

**Table 2**

| Liver fat level | Liver risk level |
| --- | --- |
| 0 to 4 | Normally |
| 4 to 7 | Suspected risk |
| 7 to 10 | Medium or high risk |

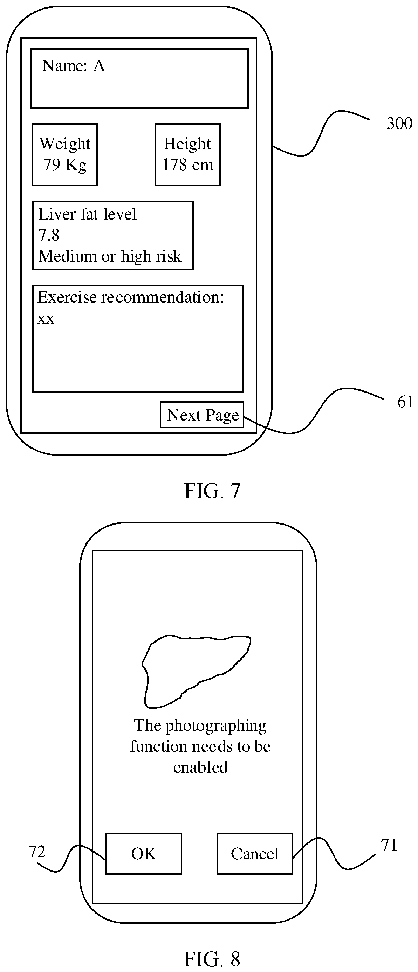

To help the to-be-measured person A learn of the liver fat level of the to-be-measured person A in time, in this solution, the electronic device 300 may present, to the to-be-measured person A, the liver fat level of the to-be-measured person A detected by the electronic device. For example, as shown in FIG. 7, the electronic device 300 may display the liver fat level of the to-be-measured person A being 7.8, and a screening result is "Medium or high risk". In addition, still refer to FIG. 7. The electronic device 300 may further display other parameters of the to-be-measured person A, for example, the body height, the body shape (not shown in the figure), and the like, and display an exercise recommendation.

In this way, in this solution, in a process of determining the liver fat level, for users of different body shapes, different level determination models may be used to calculate liver fat levels of the users, so that different processing manners are used for different people, evaluation accuracy of a liver fat level is improved, and a case in which one liver fat level evaluation manner is used for all people is avoided.

The above is an introduction to the solution for detecting a liver fat level in this solution. After the liver fat level is obtained, if a user wants a more accurate result, in this solution, a body feature parameter of the user may be further added in a detection process. The body feature parameter includes a bust circumference, a waist circumference, a hip circumference, and the like. It may be understood that the body feature parameter is a parameter that is strongly related to fat content in the liver, to improve detection precision of a liver fat level. In an example, in this case, the system for detecting a liver fat level in this solution may be the system shown in FIG. 1a. The mobile phone 12 may acquire a photo of a to-be-measured person, to determine the body feature parameter of the to-be-measured person. In addition, in this case, the system for detecting a liver fat level in this solution may be the system shown in FIG. 1b. A camera may be disposed on the smart screen 13. After the to-be-measured person determines the accurately measured liver fat level from the mobile phone 12, the mobile phone 12 may send, to the smart screen 13, an instruction for acquiring a photo of the to-be-measured person, to acquire the photo of the to-be-measured person with the camera on the smart screen 13. Subsequently, the smart screen 13 sends the photo of the to-be-measured person to the mobile phone 12, to determine the body feature parameter of the to-be-measured person by using the mobile phone 12, and obtain a more precise liver fat level. For details, refer to the following description.

The following describes in detail a solution of adding a body feature parameter of a user by scenario.

Scenario 1

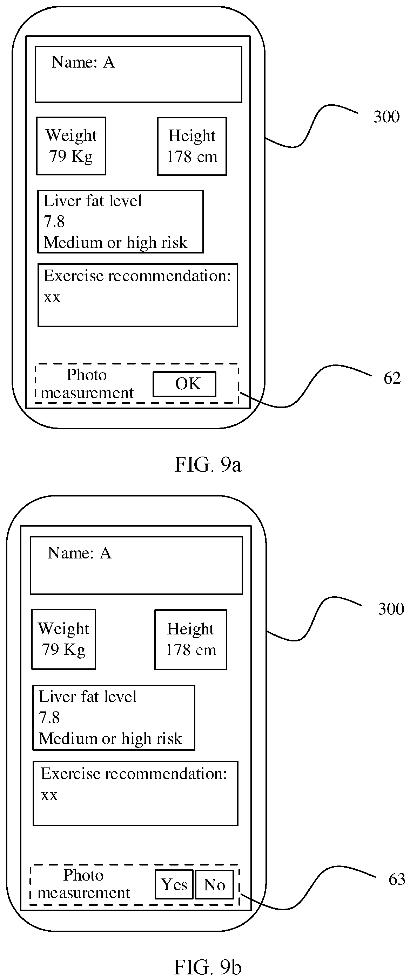

In this scenario, the electronic device 300 is a mobile phone. This scenario may be understood as an application scenario in the system shown in FIG. 1a. An application program (for example, Huawei Health) related to a liver fat level may be installed on the mobile phone. Still refer to FIG. 7. In this case, the to-be-measured person A may select a "Next Page" button in an area 61. Next, the electronic device 300 may display an interface shown in FIG. 8, to prompt the to-be-measured person A with "The phone camera needs to be turned on", and the to-be-measured person A selects whether to measure liver fat more precisely. If the to-be-measured person A selects a "Cancel" button in an area 71, precise liver fat measurement is stopped, and an interface shown in FIG. 7 is returned. If the to-be-measured person A selects an "OK" button in an area 72, a procedure of precisely measuring liver fat is performed. In addition, in addition to selecting the area 61 in FIG. 7 to display the interface shown in FIG. 8, the to-be-measured person A may further swipe on the interface shown in FIG. 7, for example, swipe from a left side to a right side of the screen of the electronic device 300, swipe from a right side to a left side of the electronic device 300, swipe from an upper side to a lower side of the electronic device 300, or swipe from a lower side to an upper side of the electronic device 300, to display the interface shown in FIG. 8. It may be understood that, in this solution, "Next Page" displayed in the area 61 in FIG. 7 may be replaced with other content. For example, as shown in FIG. 9, "Next Page" in the area 61 in FIG. 7 may be replaced with content in the area 62 in FIG. 9a, so that the to-be-measured person A selects an "OK" button. After the to-be-measured person A selects the "OK" button, the interface shown in FIG. 8 is entered. In addition, as shown in FIG. 9b, "Next Page" in the area 61 in FIG. 7 may be replaced with content in an area 63 in FIG. 9b, so that the to-be-measured person A selects "Yes" or "No". After the to-be-measured person A selects "Yes", the interface shown in FIG. 8 is entered. In an example, content in the area 63 in FIG. 9b may appear in a form of a pop-up window. After the to-be-measured person A selects "Yes", the interface shown in FIG. 8 is entered. After the to-be-measured person A selects "No", the pop-up window may be closed, in other words, content in the area 63 is no longer displayed. For example, the pop-up window may pop up after the liver fat level is detected for a period of time (for example, 3 seconds).

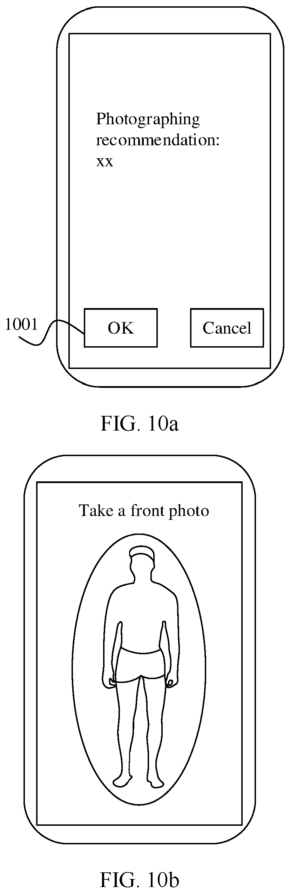

Because a whole-body image of a user needs to be photographed at a specific height during photographing, when the electronic device 300 is a mobile phone, the mobile phone needs to be fixed at a specific position. As shown in FIG. 10a, after the to-be-measured person A selects the "OK" button in the area 72 on the mobile phone shown in FIG. 8, the mobile phone may display recommendation information before photographing. The recommendation information may be "1. Place the mobile phone at a fixed position, and ensure that the mobile phone can take a clear whole-body photo. 2. Wear tight clothes. Expose the waist and abdomen. Keep the hands down. Do not stick the hands to the legs." In this way, the to-be-measured person A is prompted to make an appropriate photographing gesture, to improve measurement accuracy. Further, when getting ready, the to-be-measured person A may select an "OK" button in an area 1001 in FIG. 10a, and enter a photographing process. It may be understood that, in this solution, when selecting a button on the mobile phone, the to-be-measured person A may select the button with a tap, or may select the button by voice, or may select the button with a gesture. This may be specifically determined according to an actual case, and is not limited herein.

Next, after entering a photographing procedure, as shown in FIG. 10b, the mobile phone may first take a front photo of the to-be-measured person A. After taking the front photo of the to-be-measured person A, the mobile phone may detect whether the photo meets a requirement. If the photo meets the requirement, the mobile phone enters a next procedure. If the photo does not meet the requirement, the mobile phone retakes a front photo. For example, after the front photo of the to-be-measured person A is taken, human skeleton nodes may be selected from the taken image by using a template matching-based human skeleton key point detection algorithm (Pictorial Structure) and an object detection-based human skeleton key point detection algorithm, for example, a cascaded feature network (cascaded feature network, CFN), a regional multi-person pose estimation (regional multi-person pose estimation, RMPE), or a cascaded pyramid network (cascaded pyramid network, CPN), and the like, and a body vector is constructed. Subsequently, a preset standard limb vector is compared with an obtained limb vector, to obtain an action difference degree between the two. Next, it is determined, based on the action difference degree, whether the obtained image meets the requirement. For example, when the action difference degree falls within a preset range, it is determined that the requirement is met. When the action difference degree does not fall within the preset range, it is determined that the requirement is not met. It may be understood that the figure image in FIG. 10b is merely an example of a front image of the to-be-measured person A currently acquired by the mobile phone.

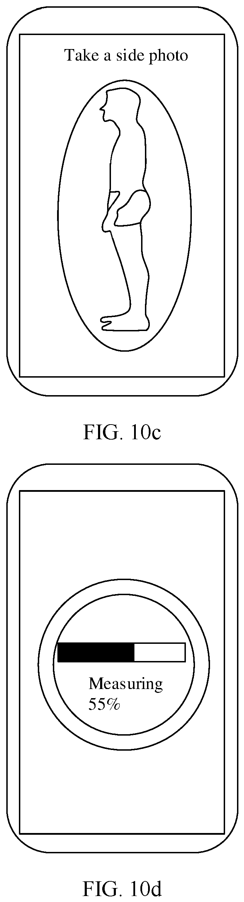

After the mobile phone detects that the taken image meets the requirement, the mobile phone may take a side photo of the to-be-measured person A, in other words, display an interface shown in FIG. 10c, to take the side photo of the to-be-measured person A. After taking the side photo of the to-be-measured person A, the mobile phone may detect whether the photo meets a requirement. For a detection method, refer to the manner of detecting a front photo. Details are not described herein again. If the photo meets the requirement, a next procedure is entered. If the photo does not meet the requirement, a side photo is retaken. It may be understood that the figure image in FIG. 10c is merely an example of a side image of the to-be-measured person A currently acquired by the mobile phone.

After the mobile phone detects that both the front photo and the side photo meet the requirements, the mobile phone may perform image segmentation on the taken front photo and side photo based on a neural network (for example, a Unet network, or the like), to extract a human body portrait. Subsequently, the mobile phone determines corresponding human body node positions, for example, the armpits, groin, navel, thigh roots, and the like, based on bone nodes and contour nodes. For example, the corresponding human body node positions may be determined by using the foregoing described human skeleton key point detection algorithm. Next, the mobile phone may determine feature information such as a chest width, a chest thickness, a waist width, a waist thickness, a hip width, a hip thickness, and the like at the human body node positions with reference to a body proportion that is of the to-be-measured person A and that is determined based on the human body portrait and the body height. Next, the mobile phone may infer, based on the determined feature information, body feature parameters such as the bust circumference, the waist circumference, and the hip circumference. For example, the waist circumference is used as an example. Because the waist circumference is an ellipse-like shape, after the waist width and the waist thickness are obtained, the waist circumference may be obtained through inference based on mathematical operations. Further, after the body feature parameter is obtained, the waist-to-hip ratio may be calculated based on the waist circumference and the hip circumference in the body feature parameter. It may be understood that the waist-to-hip ratio measured before photographing is calculated according to a formula set by experience, and in this case, the calculated waist-to-hip ratio is inaccurate. The waist-to-hip ratio measured after photographing is calculated based on the feature information of the body of a to-be-measured person, and can truly reflect a body condition of the to-be-measured person. In other words, the calculated waist-to-hip ratio is accurate.

Further, the mobile phone may input the body feature parameter such as the waist circumference or the like and the body parameters such as the BMI, the body fat ratio, and the waist-to-hip ratio of the to-be-measured person A into the determined level determination model, and may obtain the liver fat level of the to-be-measured person A after processing by the level determination model. In this case, the waist-to-hip ratio inputted into the level determination model may be a waist-to-hip ratio inferred from a photo of the to-be-measured person, or may be a waist-to-hip ratio calculated before photographing. However, to improve detection accuracy, the waist-to-hip ratio inferred from the photo of the to-be-measured person is preferably selected. In an example, after the waist-to-hip ratio of the to-be-measured person is determined by using the front-side photo and the side-side photo, the waist-to-hip ratio may be compared with a waist-to-hip ratio determined based on the body impedance. If the waist-to-hip ratios belong to the same interval during determining of the parameters required for the body shape, the level determination model determined before photographing may continue to be used. If the waist-to-hip ratios belong to different intervals during determining of the parameters required for the body shape, a body shape of the to-be-measured person A may be redetermined by using the waist-to-hip ratio determined based on the photo, and a level determination model is redetermined, and the liver fat level of the to-be-measured person A is calculated by using the redetermined level determination model. In an example, after the body shape of the to-be-measured person A is redetermined, the body shape determined before photographing may be replaced with the redetermined body shape, and the body shape is presented to the to-be-measured person A.

In this solution, after the mobile phone takes the front-side photo and the side-side photo and before the liver fat level of the to-be-measured person A is obtained, in other words, in a process of calculating the liver fat level of the to-be-measured person A, the mobile phone may display an interface shown in FIG. 10d, so that the to-be-measured person learns of processing progress. It may be understood that the parameters inputted into the level determination model may include other parameters, for example, the visceral fat mass in the torso, the fat mass of each section of the body, the body shape, the bust circumference, the hip circumference, and the like, to improve detection accuracy.

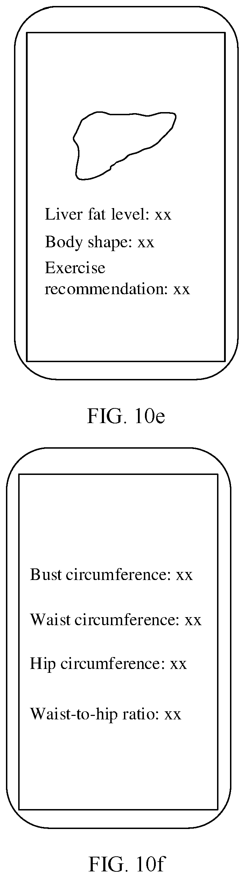

After the mobile phone calculates the liver fat level of the to-be-measured person A, as shown in FIG. 10e, the mobile phone displays "Liver fat level". In addition, to enable the to-be-measured person A to learn of the body feature parameter (that is, body size information) of the to-be-measured person A, the mobile phone may further display an interface shown in FIG. 10f, so that the to-be-measured person A can intuitively learn of parameters such as the bust circumference, the waist circumference, the hip circumference, and the waist-to-hip ratio of the to-be-measured person A. It may be understood that a display sequence of the interfaces shown in FIG. 10e and FIG. 10f is determined as required, and is not limited herein.

Scenario 2

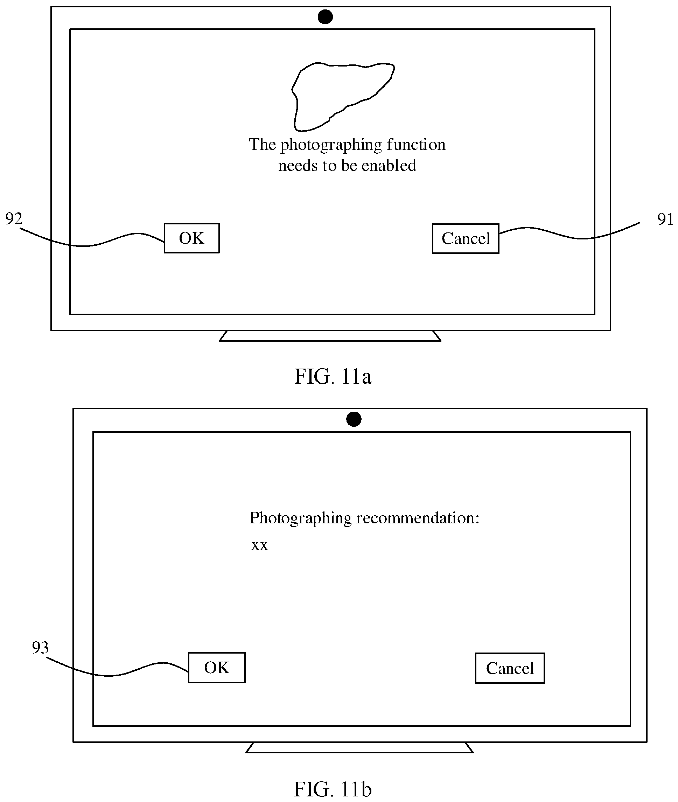

In this scenario, the electronic device 300 is a large screen at a fixed position, for example, a smart screen. An image acquisition apparatus (for example, a camera) is configured on the large screen, to acquire an image of a to-be-measured person. In addition, an application program (for example, Huawei Health) related to a liver fat level may be installed on the large screen. This scenario is equivalent to replacing the mobile phone 12 in FIG. 1a with a large screen. In this scenario, interaction is performed between the large screen and a body fat scale. A connection may be established between the large screen and the body fat scale via Bluetooth. Still refer to FIG. 7. In this case, the to-be-measured person A may tap the area 61 in which a "Fatty liver risk level" is located. For example, a color of the area 61 in FIG. 7 may be different from a color of another area in FIG. 6, so that the to-be-measured person A may learn that the area 61 is tappable. For example, the color of the another area in FIG. 7 may be gray or white, and the color of the area 61 may be green or blue. Next, the electronic device 300 may display an interface shown in FIG. 11a, to prompt the to-be-measured person A with "The large-screen photographing function needs to be enabled. ", and the to-be-measured person A selects whether to measure liver fat more precisely. If the to-be-measured person A selects a "Cancel" button in an area 91, precise liver fat measurement is stopped, and an interface shown in FIG. 7 is returned. If the to-be-measured person A selects an "OK" button in an area 92, a procedure of precisely measuring liver fat is performed. In addition, in addition to selecting the area 61 in FIG. 7 to display the interface shown in FIG. 11a, the to-be-measured person A may further swipe on the interface shown in FIG. 7, for example, swipe from a left side to a right side of the screen of the electronic device 300, swipe from a right side to a left side of the electronic device 300, swipe from an upper side to a lower side of the electronic device 300, or swipe from a lower side to an upper side of the electronic device 300, to display the interface shown in FIG. 11a. It may be understood that, in this solution, "Next Page" displayed in the area 61 in FIG. 7 may be replaced with other content. For example, as shown in FIG. 9, "Next Page" in the area 61 in FIG. 7 may be replaced with content in the area 62 in FIG. 9, so that the to-be-measured person A selects "Yes" or "No". After the to-be-measured person A selects "Yes", the interface shown in FIG. 11a is entered.

After the to-be-measured person A selects an "OK" button on the large screen, as shown in FIG. 11b, the large screen may display recommendation information before photographing. The recommendation information may be "Wear tight clothes. Expose the waist and abdomen. Keep the hands down. Do not stick the hands to the legs." In this way, the to-be-measured person A is prompted to make an appropriate photographing gesture, to improve measurement accuracy. Further, when getting ready, the to-be-measured person A may select an "OK" button in an area 93 in FIG. 11b, and enter a photographing process. It may be understood that, in this solution, when selecting a button on the large screen, the to-be-measured person A may select the button with a tap, or may select the button by voice, or may select the button with a gesture. This may be specifically determined according to an actual case, and is not limited herein.

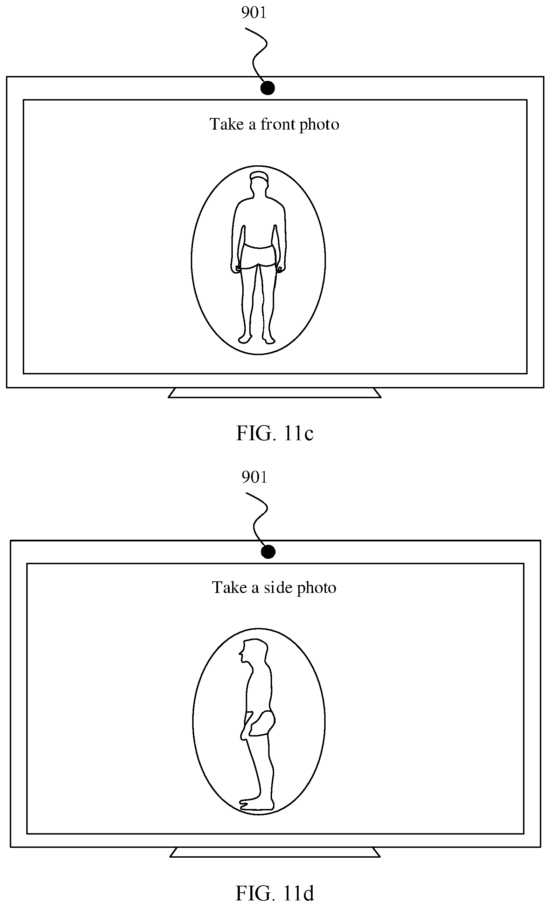

Next, after entering a photographing procedure, as shown in FIG. 11c, the large screen may first take a front photo of the to-be-measured person A by using a camera 901. After taking the front photo of the to-be-measured person A, the large screen may detect whether the photo meets a requirement. If the photo meets the requirement, a next procedure is entered. If the photo does not meet the requirement, a front photo is retaken by using the camera 901. For a manner of detecting whether a photo meets a requirement, refer to descriptions in the foregoing "Scenario 1", and details are not described herein again. It may be understood that the figure image in FIG. 11c is merely an example of a front image of the to-be-measured person A currently acquired by the large screen.

After the large screen detects that the taken image meets the requirement, the large screen takes a side photo of the to-be-measured person A, in other words, may display an interface shown in FIG. 11d, and take the side photo of the to-be-measured person A by using the camera 901. After taking the side photo of the to-be-measured person A, the large screen may detect whether the photo meets a requirement. If the photo meets the requirement, a next procedure is entered. If the photo does not meet the requirement, a front photo is retaken by using the camera 901. It may be understood that the figure image in FIG. 11d is merely an example of a front image of the to-be-measured person A currently acquired by the large screen.

After the large screen detects that both the front photo and the side photo meet the requirements, the large screen may perform image segmentation on the taken front photo and side photo based on a neural network (for example, a Unet network), to extract a human body portrait. Subsequently, the large screen determines corresponding human body node positions, for example, the armpits, groin, navel, thigh roots, and the like, based on bone nodes and contour nodes. Next, the large screen may determine feature information such as a chest width, a chest thickness, a waist width, a waist thickness, a hip width, a hip thickness, a thigh width, a thigh thickness, and the like at the human body node positions with reference to the human body portrait and the body height. Next, the large screen may infer, based on the determined feature information, body feature parameters such as the bust circumference, the waist circumference, and the hip circumference. For example, the waist circumference is used as an example. Because the waist circumference is an ellipse-like shape, after the waist width and the waist thickness are obtained, the waist circumference may be obtained through inference based on mathematical operations. Further, after the body feature parameter is obtained, the waist-to-hip ratio may be calculated based on the waist circumference and the hip circumference in the body feature parameter. It may be understood that the waist-to-hip ratio measured before photographing is calculated according to a formula set by experience, and in this case, the calculated waist-to-hip ratio is inaccurate. The waist-to-hip ratio measured after photographing is calculated based on the feature information of the body of a to-be-measured person, and can truly reflect a body condition of the to-be-measured person. In other words, the calculated waist-to-hip ratio is accurate. It may be understood that the parameters inputted into the level determination model may include other parameters, for example, the visceral fat mass in the torso, the fat mass of each section of the body, the body shape, the bust circumference, the hip circumference, and the like, to improve detection accuracy.

Further, the large screen may input the body feature parameter such as the waist circumference or the like and the body parameters such as the BMI, the body fat ratio, and the waist-to-hip ratio of the to-be-measured person A into the determined level determination model, and may obtain the liver fat level of the to-be-measured person A after processing by the level determination model. In this case, the waist-to-hip ratio inputted into the level determination model may be a waist-to-hip ratio inferred from a photo of the to-be-measured person, or may be a waist-to-hip ratio calculated before photographing. However, to improve detection accuracy, the waist-to-hip ratio inferred from the photo of the to-be-measured person is preferably selected. In an example, after the waist-to-hip ratio of the to-be-measured person is determined by using the front-side photo and the side-side photo, the waist-to-hip ratio may be compared with a waist-to-hip ratio determined based on the body impedance. If the waist-to-hip ratios belong to the same interval during determining of the parameters required for the body shape, the level determination model determined before photographing may continue to be used. If the waist-to-hip ratios belong to different intervals during determining of the parameters required for the body shape, a body shape of the to-be-measured person A may be redetermined by using the waist-to-hip ratio determined based on the photo, and a level determination model is redetermined, and the liver fat level of the to-be-measured person A is calculated by using the redetermined level determination model. In an example, after the body shape of the to-be-measured person A is redetermined, the body shape determined before photographing may be replaced with the redetermined body shape, and the body shape is presented to the to-be-measured person A.

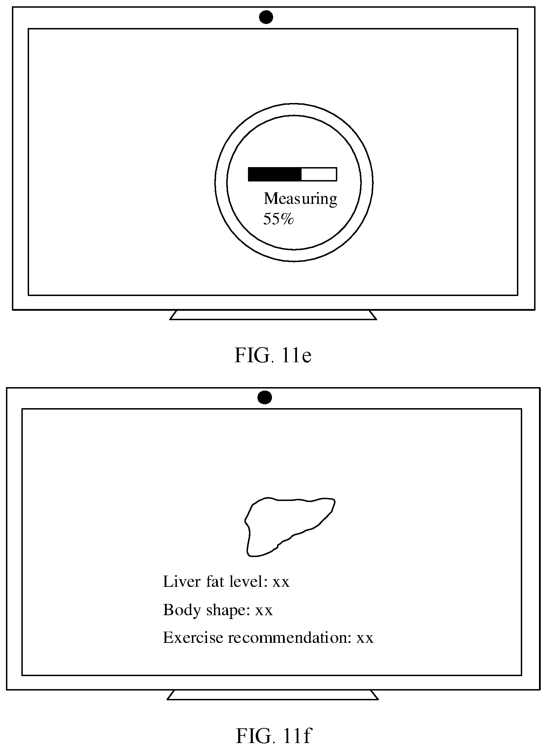

In this solution, after the large screen takes the front-side photo and the side-side photo and before the liver fat level of the to-be-measured person A is obtained, in other words, in a process of calculating the liver fat level of the to-be-measured person A, the large screen may display an interface shown in FIG. 11e, so that the to-be-measured person learns of processing progress.

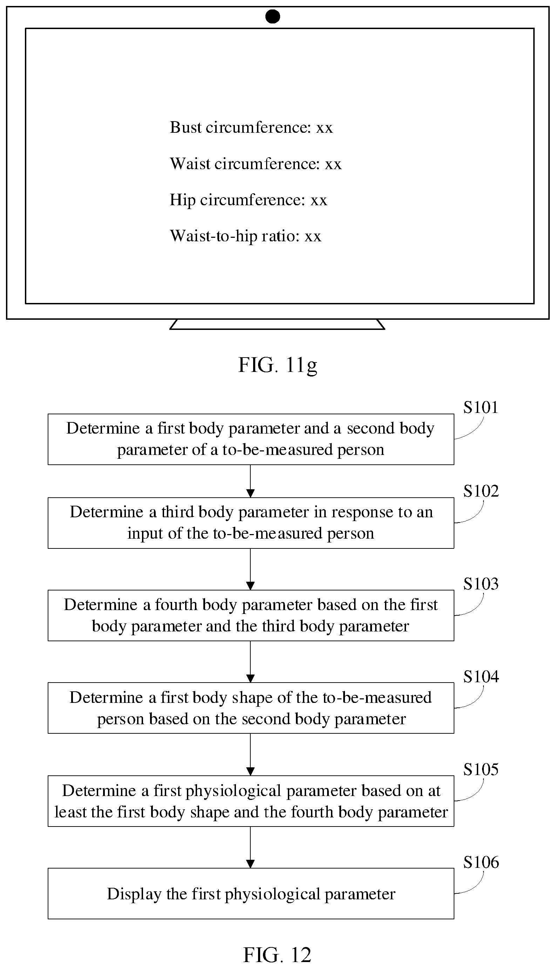

After the large screen calculates the liver fat level of the to-be-measured person A, as shown in FIG. 11f, the large screen displays "Liver fat level". In addition, to enable the to-be-measured person A to learn of the body feature parameter (that is, body size information) of the to-be-measured person A, the large screen may further display an interface shown in FIG. 11g, so that the to-be-measured person A can intuitively learn of parameters such as the bust circumference, the waist circumference, the hip circumference, and the waist-to-hip ratio of the to-be-measured person A. It may be understood that a display sequence of the interfaces shown in FIG. 11f and FIG. 11g is determined as required, and is not limited herein.

It should be noted that, in this solution, prompt information of operation procedures in FIG. 11a to FIG. 11g may be replaced with other prompt information, and the prompt information may prompt a user to perform an action that is the same as or similar to a standard action, which is not limited herein. In addition, prompt information in another figure may be replaced with other prompt information, which is not limited herein.

Scenario 3

The scenario is an application scenario in the system shown in FIG. 1b. An application program (for example, Huawei Health) related to a liver fat level may be installed on the mobile phone 12. In this scenario, after the mobile phone 12 displays a liver fat level (that is, the interface shown in FIG. 7, and in this case, the electronic device 300 may be the mobile phone 12), the to-be-measured person A may tap the area 61 in which "Next Page" is located in FIG. 7. Then, as shown in FIG. 10a, the mobile phone 12 may display recommendation information before detection. The recommendation information may be "1. The large-screen photographing function needs to be enabled. Ensure that the large screen is turned on. 2. Wear tight clothes. Expose the waist and abdomen. Keep the hands down. Do not stick the hands to the legs." The large screen may be understood as smart screen 13. If the to-be-measured person A selects a "Cancel" button, precise liver fat measurement is stopped, and an interface shown in FIG. 7 is returned. If the to-be-measured person A selects an "OK" button, the mobile phone 12 sends a photographing instruction to the smart screen 13. It may be understood that, in this solution, when selecting a button on the mobile phone, the to-be-measured person A may select the button with a tap, or may select the button by voice, or may select the button with a gesture. This may be specifically determined according to an actual case, and is not limited herein.

After receiving the photographing instruction sent by the mobile phone 12, the smart screen 13 may turn on a camera 1201 configured on the smart screen, to enter a photographing procedure. In the photographing procedure, the smart screen 13 may first take a front photo of the to-be-measured person A by using the camera 1201 on the smart screen. After taking the front photo of the to-be-measured person A, the smart screen 13 may send the photo to the mobile phone 12, so that the mobile phone 12 detects whether the photo meets a requirement. When detecting that the photo meets the requirement, the mobile phone 12 sends, to the smart screen 13, an instruction for entering a next procedure. When detecting that the photo does not meet the requirement, the mobile phone 12 sends, to the smart screen 13, a rephotographing instruction, to retake the front photo by using the camera 1201. For a manner of detecting, by the mobile phone 12, whether a photo meets a requirement, refer to descriptions in the foregoing "Scenario 1", and details are not described herein again.

After the smart screen 13 receives the instruction that is sent by the mobile phone 12 and that is for entering the next procedure, the smart screen 13 may take a side photo of the to-be-measured person A by using the camera 1201. After taking the side photo of the to-be-measured person A, the smart screen 13 may send the photo to the mobile phone 12, so that the mobile phone 12 detects whether the photo meets a requirement. When detecting that the photo meets the requirement, the mobile phone 12 sends, to the smart screen 13, an instruction for indicating that photographing ends. When detecting that the photo does not meet the requirement, the mobile phone 12 sends, to the smart screen 13, a rephotographing instruction, to retake the side photo by using the camera 1201.

After receiving the instruction that is sent by the mobile phone 12 and that is used for indicating that photographing ends, the smart screen 13 may turn off the camera, and end photographing.

Further, after the mobile phone 12 receives the front photo and the side photo of the to-be-measured person A that are sent by the smart screen 13 and detects that both the front photo and the side photo meet the requirements, the mobile phone 12 may perform image segmentation on the taken front photo and side photo based on a neural network (for example, a Unet network), to extract a human body portrait. Subsequently, the mobile phone 12 determines corresponding human body node positions, for example, the armpits, groin, navel, thigh roots, and the like, based on bone nodes and contour nodes. Next, the mobile phone 12 may determine feature information such as a chest width, a chest thickness, a waist width, a waist thickness, a hip width, a hip thickness, a thigh width, a thigh thickness, and the like at the human body node positions with reference to the human body portrait and the body height. Next, the mobile phone 12 may infer, based on the determined feature information, body feature parameters such as the bust circumference, the waist circumference, and the hip circumference. For example, the waist circumference is used as an example. Because the waist circumference is an ellipse-like shape, after the waist width and the waist thickness are obtained, the waist circumference may be obtained through inference based on mathematical operations.

Further, the mobile phone 12 may input the body feature parameter such as the waist circumference or the like and the body parameters such as the BMI, the body fat ratio, and the waist-to-hip ratio of the to-be-measured person A into the determined level determination model, and may obtain the liver fat level of the to-be-measured person A after processing by the level determination model. In an example, after the waist-to-hip ratio of the to-be-measured person is determined by using the front-side photo and the side-side photo, the waist-to-hip ratio may be compared with a waist-to-hip ratio determined based on the body impedance. If the waist-to-hip ratios belong to the same interval during determining of the parameters required for the body shape, the level determination model determined before photographing may continue to be used. If the waist-to-hip ratios belong to different intervals during determining of the parameters required for the body shape, a body shape of the to-be-measured person A may be redetermined by using the waist-to-hip ratio determined based on the photo, and a level determination model is redetermined, and the liver fat level of the to-be-measured person A is calculated by using the redetermined level determination model. In an example, after the body shape of the to-be-measured person A is redetermined, the body shape determined before photographing may be replaced with the redetermined body shape, and the body shape is presented to the to-be-measured person A.

In this solution, after the mobile phone 12 obtains the front-side photo and the side-side photo of the to-be-measured person A and before the liver fat level of the to-be-measured person A is obtained, in other words, in a process of calculating the liver fat level of the to-be-measured person A, the mobile phone 12 may display an interface shown in FIG. 10d, so that the to-be-measured person learns of processing progress. It may be understood that the parameters inputted into the level determination model may include other parameters, for example, the visceral fat mass in the torso, the fat mass of each section of the body, the body shape, the bust circumference, the hip circumference, and the like, to improve detection accuracy.

After the mobile phone 12 calculates the liver fat level of the to-be-measured person A, as shown in FIG. 10e, the mobile phone 12 displays "Liver fat level". In addition, to enable the to-be-measured person A to learn of the body feature parameter (that is, body size information) of the to-be-measured person A, the mobile phone 12 may further display an interface shown in FIG. 10f, so that the to-be-measured person A can intuitively learn of parameters such as the bust circumference, the waist circumference, the hip circumference, and the waist-to-hip ratio of the to-be-measured person A. It may be understood that a display sequence of the interfaces shown in FIG. 10e and FIG. 10f is determined as required, and is not limited herein. In addition, the mobile phone 12 may send the measured liver fat level and/or form feature parameter to the smart screen 13, for example, to the smart screen 13 through screen casting, for display on the smart screen 13.

Therefore, in a process of detecting a liver fat level, parameters such as a body feature parameter and a body shape of a to-be-measured person are combined, so that while different processing manners are used for different people, evaluation accuracy of a liver fat level is further improved, and a case in which one liver fat level evaluation manner is used for all people is avoided.

The following describes, based on the foregoing described solution of detecting a liver fat level, a method for detecting physiological parameters provided in an embodiment of this application. It may be understood that the method is proposed based on the foregoing described solution of detecting a liver fat level. For a part or all of content of the method, refer to the foregoing description of the solution of detecting a liver fat level.

FIG. 12 is a schematic flowchart of a method for measuring physiological parameters according to an embodiment of this application. As shown in FIG. 12, the method may include the following steps.

Step 101: Determine a first body parameter and a second body parameter of a to-be-measured person.

In this solution, both the first body parameter and the second body parameter may be measured by the first electronic device. The first electronic device may have at least eight electrodes. The second body parameter may be measured based on at least eight electrodes of the first electronic device. For example, the second body parameter may be measured by the first electronic device by controlling the at least eight electrodes to generate at least two electrical signals with different frequencies. In this way, the second body parameter is measured by using electrical signals with different frequencies, to improve precision of subsequent detection. For example, the first electronic device may be the body fat scale 11 shown in FIG. 1a.

In an example, the first body parameter may be a body weight, the second body parameter may be a body impedance or raw data (for example, a voltage, a current, or the like) required for calculating a body impedance. In an example, the body impedance may include an arms-related impedance, a legs-related impedance, and a torso-related impedance. For example, the arms-related impedance may include a two-arm impedance, a left-arm impedance, a right-arm impedance, and the like. The legs-related impedance may include a two-leg impedance, a left-leg impedance, a right-leg impedance, and the like. The torso-related impedance may include a torso impedance, another impedance including a torso impedance, and the like.

Step 102: Determine a third body parameter in response to an input of the to-be-measured person.

For example, the third body parameter may be a body height.

Step 103: Determine a fourth body parameter based on the first body parameter and the third body parameter.

For example, the fourth body parameter may be a body mass index BMI. When the first body parameter is the body weight and the third body parameter is the body height, the fourth body parameter may be the BMI. A formula for determining the BMI: The body mass index is $BMI = W/H^2$. W is the body weight, and H is the body height.

Step 104: Determine a first body shape of the to-be-measured person based on the second body parameter.

In an example, during determining of the first body shape of the to-be-measured person, a fifth body parameter may be first determined based on the second body parameter. Then, an interval to which the fifth body parameter belongs is determined. If the fifth body parameter belongs to a first interval, the first body shape of the to-be-measured person is determined based on the fourth body parameter. If the fifth body parameter belongs to a second interval, the first body shape of the to-be-measured person is determined based on a sixth body parameter, where the sixth body parameter is obtained based on the second body parameter. For example, the fifth body parameter may be a waist-to-hip ratio, and the sixth body parameter may be muscle mass, fat mass, or the like of the arms of the to-be-measured person. When the second body parameter is body impedance, fat mass of each section of the body of the to-be-measured person may be first determined based on the body impedance by using the foregoing Formula 3, and then the waist-to-hip ratio is determined based on the fat mass by using the foregoing Formula 4. Next, body parameters to be used are selected for the waist-to-hip ratio, and finally the body shape is determined by using the selected body parameters. For example, the selected body parameters may be the BMI, the muscle mass, the fat mass, and the like. For a method for calculating the muscle mass, refer to the description in the foregoing Formula 5.

Step 105: Determine a first physiological parameter based on at least the first body shape and the fourth body parameter.

In this solution, a predetermined correspondence between a body shape and a detection model may be queried based on the first body shape, to determine a detection model corresponding to the first body shape, where different body shapes correspond to different detection models in the correspondence; and at least the fourth body parameter may be inputted into the detection model corresponding to the first body shape, to determine the first physiological parameter. Therefore, a detection model that matches the body shape of the to-be-measured person is determined based on the body shape of the to-be-measured person, and the physiological parameters of the to-be-measured person are detected by using the detection model, so that physiological parameters are detected for users of different body shapes by using different detection models, to improve accuracy of the physiological parameters. For example, the detection model may be the foregoing described level determination model, and the correspondence may be shown in the foregoing Table 1. For example, the first physiological parameter may be a liver fat level.

Step 106: Display the first physiological parameter.

In this solution, after the first physiological parameter of the to-be-measured person is determined, the first physiological parameter may be displayed, so that the to-be-measured person can intuitively view the physiological parameter of the to-be-measured person. For example, when the first physiological parameter is the liver fat level, the first physiological parameter may be displayed on the interface shown in FIG. 7.

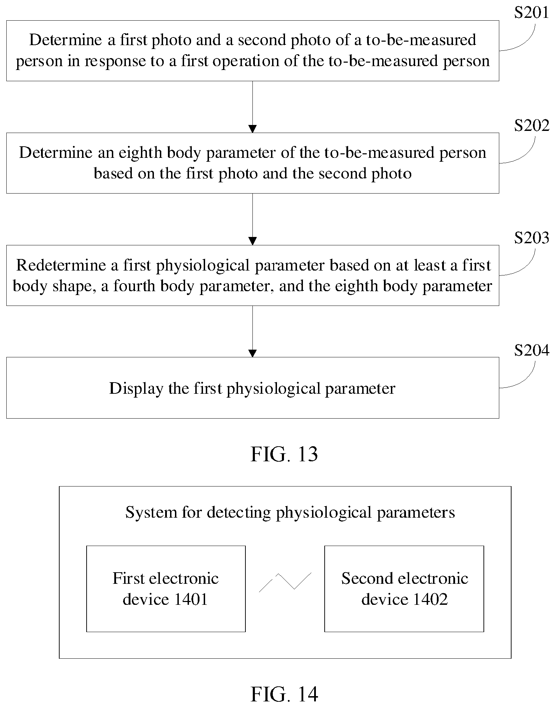

In an example, after the first physiological parameter is displayed, the first physiological parameter of the to-be-measured person may be further precisely measured. Specifically, as shown in FIG. 13, the method includes the following steps.

Step 201: Determine a first photo and a second photo of the to-be-measured person in response to a first operation of the to-be-measured person.

The to-be-measured person may perform the first operation, and the first operation may be an operation of accurately measuring the first physiological parameter. Subsequently, the first photo and the second photo of the to-be-measured person may be determined in response to the first operation. In this solution, the first photo is taken when the to-be-measured person performs a first preset action, and the second photo is taken when the to-be-measured person performs a second preset action.

For example, when the first physiological parameter is the liver fat level, the first operation may be a tap operation performed by the to-be-measured person in the area 61 in FIG. 7. The first photo may be a front photo of the to-be-measured person, and the second photo may be a side photo of the to-be-measured person. For a manner of obtaining the front photo and the side photo, refer to the descriptions in the foregoing Scenarios 1 to 3. Details are not described herein again.

In an example, the determining a first photo and a second photo of the to-be-measured person may specifically include: determining an action difference degree between an action of the to-be-measured person in a currently obtained target photo and a target preset action, and determining that the action difference degree falls within a preset range, where the target photo is the first photo, and the target preset action is the first preset action; or the target photo is the second photo, and the target preset action is the second preset action. Therefore, when the to-be-measured person performs a preset action, an acquired photo is used for detection, to improve detection accuracy of the physiological parameters. For example, an action difference degree may be detected by using a template matching-based human skeleton key point detection algorithm (Pictorial Structure) and an object detection-based human skeleton key point detection algorithm, for example, a cascaded feature network (cascaded feature network, CFN), a regional multi-person pose estimation (regional multi-person pose estimation, RMPE), or a cascaded pyramid network (cascaded pyramid network, CPN), and the like. For details, refer to the foregoing descriptions.

Step 202: Determine an eighth body parameter of the to-be-measured person based on the first photo and the second photo.

After the first photo and the second photo are determined, the eighth body parameters of the to-be-measured person may be determined based on the first photo and the second photo. The eighth body parameter is a parameter strongly related to the first physiological parameter. For example, the eighth body parameter may be a waist circumference. For a manner of determining the waist circumference, refer to descriptions in the foregoing Scenarios 1 to 3. Details are not described herein again.

Step 203: Redetermine the first physiological parameter based on at least the first body shape, the fourth body parameter, and the eighth body parameter.

After the eighth parameter is determined, a predetermined correspondence between a body shape and a detection model may be queried based on the first body shape, to determine a detection model corresponding to the first body shape, where different body shapes correspond to different detection models in the second correspondence; and at least the fourth body parameter and the eighth body parameter may be inputted into the detection model corresponding to the first body shape, to redetermine the first physiological parameter. For example, the detection model may be the foregoing described level determination model, and the correspondence may be shown in the foregoing Table 1. For example, the first physiological parameter may be a liver fat level.

In an example, before the fourth body parameter and the eighth body parameter are inputted into the detection model corresponding to the first body shape, a ninth body parameter of the to-be-measured person may further be determined based on the first photo and the second photo; and a second body shape of the to-be-measured person may be determined based on the eighth body parameter and the ninth body parameter. For example, the ninth body parameter may be a hip circumference. Because the body shape of the to-be-measured person can be accurately presented in a photo, the body shape of the to-be-measured person can be accurately determined by using a photo of the to-be-measured person, to improve detection accuracy of the body shape. For a manner of determining the hip circumference, refer to descriptions in the foregoing Scenarios 1 to 3. Details are not described herein again.

In an example, if the second body shape is inconsistent with the first body shape, the correspondence may be queried based on the second body shape, to determine a detection model corresponding to the second body shape, and the detection model corresponding to the second body shape may be selected to redetermine the first physiological parameter. Therefore, the body shape of the to-be-measured person detected by using a photo is used as a reference, and the physiological parameters of the to-be-measured person are detected, to improve detection accuracy of the physiological parameters.

Step 204: Display the first physiological parameter.

After the first physiological parameter is determined, the redetermined first physiological parameter may be displayed. For example, a redisplayed first physiological parameter may be displayed on an interface shown in FIG. 10e. In addition, the redetermined body shape of the to-be-measured person may also be displayed.

It should be noted that the method provided in FIG. 12 may be performed by the first electronic device, or may be executed by a second electronic device. When the method is performed by the second electronic device, the second electronic device may be communicatively connected to the first electronic device. When the second electronic device determines the first body parameter and the second body parameter of the to-be-measured person, the second electronic device may receive the first body parameter and the second body parameter that are sent by the first electronic device. Therefore, the physiological parameters of the to-be-measured person are detected by using a plurality of electronic devices. For example, the first electronic device may be the body fat scale 11 shown in FIG. 1a, and the second electronic device may be the mobile phone 12 shown in FIG. 1a.

The following describes, based on the foregoing described solution of detecting a liver fat level, a system for detecting physiological parameters provided in an embodiment of this application. It may be understood that the system is proposed based on the foregoing described solution of detecting a liver fat level. For a part or all of content of the method, refer to the foregoing description of the solution of detecting a liver fat level.

FIG. 14 is a schematic diagram of an architecture of a system for measuring physiological parameters according to an embodiment of this application. As shown in FIG. 14, the system includes a first electronic device 1401 and a second electronic device 1402. The first electronic device 1401 is communicatively connected to the second electronic device 1402, and the first electronic device 1401 has at least eight electrodes.

The first electronic device 1401 may be configured to: determine a first body parameter and a second body parameter of a to-be-measured person, and send the first body parameter and the second body parameter to the second electronic device 1402, where the second body parameter is measured based on the at least eight electrodes. The second electronic device 1402 may be configured to determine a third body parameter in response to an input of the to-be-measured person. The second electronic device 1402 may be further configured to: in response to receiving the first body parameter and the second body parameter, determine a fourth body parameter based on the first body parameter and the third body parameter. The second electronic device 1402 may be further configured to determine a first body shape of the to-be-measured person based on the second body parameter. The second electronic device 1402 may be further configured to: determine a first physiological parameter based on at least the first body shape and the fourth body parameter, and display the first physiological parameter.

For example, the first electronic device 1401 may be the body fat scale 11 shown in FIG. 1a, and the second electronic device 1402 may be the mobile phone 12 shown in FIG. 1a.

In an example, the second electronic device 1402 may be further configured to:

determine a fifth body parameter based on the second body parameter; and
if the fifth body parameter belongs to a first interval, determine the first body shape of the to-be-measured person based on the fourth body parameter; and
if the fifth body parameter belongs to a second interval, determine the first body shape of the to-be-measured person based on a sixth body parameter, where the sixth body parameter is obtained based on the second body parameter.

In an example, the second electronic device 1402 may be further configured to:
determine a seventh body parameter based on the second body parameter, and determine the fifth body parameter based on the seventh body parameter.

In an example, the second electronic device 1402 may be further configured to:

query a predetermined correspondence between a body shape and a detection model based on the first body shape, to determine a detection model corresponding to the first body shape, where different body shapes correspond to different detection models in the correspondence; and
input at least the fourth body parameter into the detection model corresponding to the first body shape, to determine the first physiological parameter.

In an example, after displaying the first physiological parameter, the second electronic device 1402 may be further configured to:

determine a first photo and a second photo of the to-be-measured person in response to a first operation of the to-be-measured person, where the first photo is taken when the to-be-measured person performs a first preset action, and the second photo is taken when the to-be-measured person performs a second preset action;
determine an eighth body parameter of the to-be-measured person based on the first photo and the second photo;
redetermine the first physiological parameter based on at least the first body shape, the fourth body parameter, and the eighth body parameter; and
display the first physiological parameter.

In an example, the second electronic device 1402 may be further configured to: query a predetermined correspondence between a body shape and a detection model based on the first body shape, to determine a detection model corresponding to the first body shape, where different body shapes correspond to different detection models in the second correspondence; and input at least the fourth body parameter and the eighth body parameter into the detection model corresponding to the first body shape, to determine the first physiological parameter.

In an example, before inputting the fourth body parameter and the eighth body parameter into the detection model corresponding to the first body shape, the second electronic device 1402 may be further configured to: determine a ninth body parameter of the to-be-measured person based on the first photo and the second photo; and determine a second body shape of the to-be-measured person based on the eighth body parameter and the ninth body parameter.

In an example, the second electronic device 1402 may be further configured to: when the second body shape is inconsistent with the first body shape, query the correspondence based on the second body shape, to determine a detection model corresponding to the second body shape, and select the detection model to determine the first physiological parameter.

In an example, the second electronic device 1402 may be further configured to: determine an action difference degree between an action of the to-be-measured person in a currently obtained target photo and a target preset action, and determine that the action difference degree falls within a preset range.

The target photo is the first photo, and the target preset action is the first preset action; or the target photo is the second photo, and the target preset action is the second preset action.

In an example, the second electronic device 1402 may be further configured to display a body shape of the to-be-measured person.

In an example, the first electronic device 1401 may be further configured to: control the at least eight electrodes to generate at least two electrical signals with different frequencies; and separately determine body parameters measured based on the electrical signals with the frequencies, to obtain the second body parameter. For example, the first electronic device 1401 may control the eight electrodes to generate 50 kHz and 250 kHz electrical signals.

It should be understood that the second electronic device may be configured to perform the method in the foregoing embodiment, and an implementation principle and a technical effect of the second electronic device are similar to those described in the foregoing method. For a working process of the second electronic device, refer to a corresponding process in the foregoing method. Details are not described herein again.

It may be understood that, the processor in embodiments of this application may be a central processing unit (central processing unit, CPU), may be another general-purpose processor, a digital signal processor (digital signal processor, DSP), an application-specific integrated circuit (application-specific integrated circuit, ASIC), a field programmable gate array (field programmable gate array, FPGA), another programmable logic device, a transistor logic device, a hardware component, or any combination thereof. The general purpose processor may be a microprocessor, or may be any conventional processor.

The method steps in embodiments of this application may be implemented in a hardware manner, or may be implemented in a manner of executing software instructions by the processor. The software instructions may include corresponding software modules. The software modules may be stored in a random access memory (random access memory, RAM), a flash memory, a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), a register, a hard disk, a removable hard disk, a CD-ROM, or any other form of storage medium well-known in the art. For example, a storage medium is coupled to a processor, so that the processor can read information from the storage medium and write information into the storage medium. Certainly, the storage medium may be a component of the processor. The processor and the storage medium may be disposed in an ASIC.

All or some of the foregoing embodiments may be implemented by software, hardware, firmware, or any combination thereof. When software is used to implement embodiments, all or a part of embodiments may be implemented in a form of a computer program product. The computer program product includes one or more computer instructions. When the computer program instructions are loaded and executed on a computer, the procedure or functions according to embodiments of this application are all or partially generated. The computer may be a general purpose computer, a dedicated computer, a computer network, or another programmable apparatus. The computer instructions may be stored in a computer-readable storage medium, or may be transmitted by using the computer-readable storage medium. The computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (solid-state drive, SSD)), or the like.

It may be understood that various numbers in embodiments of this application are merely used for differentiation for ease of description, and are not used to limit the scope of embodiments of this application.

**Claims**

1. A computer implemented method for measuring physiological parameters, wherein the method comprises:

   determining (S101) a first body parameter and a second body parameter of a to-be-measured person, wherein the first body parameter is a body weight and the second body parameter is a body impedance or raw data required for calculating a body impedance, and wherein the first body parameter and the second body parameter are measured by a first electronic device, and the second body parameter is measured based on at least eight electrodes of the first electronic device;
   determining (S102) a third body parameter in response to an input of the to-be-measured person, wherein the third body parameter is a body height;
   determining (S103) a fourth body parameter based on the first body parameter and the third body parameter, wherein the fourth body parameter is a body mass index, BMI;
   determining (S104) a first body shape of the to-be-measured person based on the second body parameter;
   determining (S105) a first physiological parameter based on at least the first body shape and the fourth body parameter, wherein the first physiological parameter includes a liver fat level; and
   displaying (S106) the first physiological parameter.

2. The method according to claim 1, wherein the determining a first body shape of the to-be-measured person based on the second body parameter specifically comprises:

determining a fifth body parameter based on the second body parameter, wherein the fifth body parameter is a waist-to-hip ratio;

if the fifth body parameter belongs to a first interval, determining the first body shape of the to-be-measured person based on the fourth body parameter; and

if the fifth body parameter belongs to a second interval, determining the first body shape of the to-be-measured person based on a sixth body parameter, wherein the sixth body parameter is obtained based on the second body parameter, and wherein the sixth body parameter includes on or more of: muscle mass, fat mass of arms of the to-be-measured person.

3. The method according to claim 1 or 2, wherein the determining a first physiological parameter based on at least the first body shape and the fourth body parameter specifically comprises:

querying a predetermined correspondence between a body shape and a detection model based on the first body shape, to determine a detection model corresponding to the first body shape, wherein different body shapes correspond to different detection models in the correspondence; and

inputting at least the fourth body parameter into the detection model corresponding to the first body shape, to determine the first physiological parameter.

4. The method according to claim 1, wherein after the displaying the first physiological parameter, the method further comprises:

determining a first photo and a second photo of the to-be-measured person in response to a first operation of the to-be-measured person, wherein the first photo is taken when the to-be-measured person performs a first preset action, and the second photo is taken when the to-be-measured person performs a second preset action;

determining an eighth body parameter of the to-be-measured person based on the first photo and the second photo, wherein the eighth body parameter is a waist circumference;

redetermining the first physiological parameter based on at least the first body shape, the fourth body parameter, and the eighth body parameter; and

displaying the first physiological parameter.

5. The method according to claim 4, wherein the redetermining the first physiological parameter based on at least the first body shape, the fourth body parameter, and the eighth body parameter specifically comprises:

querying a predetermined correspondence between a body shape and a detection model based on the first body shape, to determine a detection model corresponding to the first body shape, wherein different body shapes correspond to different detection models in the second correspondence; and

inputting at least the fourth body parameter and the eighth body parameter into the detection model corresponding to the first body shape, to redetermine the first physiological parameter.

6. The method according to claim 5, wherein before the inputting at least the fourth body parameter and the eighth body parameter into the detection model corresponding to the first body shape, the method further comprises:

determining a ninth body parameter of the to-be-measured person based on the first photo and the second photo, wherein the ninth body parameter is a hip circumference; and

determining a second body shape of the to-be-measured person based on the eighth body parameter and the ninth body parameter.

7. The method according to claim 6, wherein the method further comprises:

if the second body shape is inconsistent with the first body shape,
querying the correspondence based on the second body shape, to determine a detection model corresponding to the second body shape, and selecting the detection model corresponding to the second body shape to redetermine the first physiological parameter.

8. The method according to any one of claims 4 to 7, wherein the determining a first photo and a second photo of the to-be-measured person specifically comprises:

determining an action difference degree between an action of the to-be-measured person in a currently obtained

24

target photo and a target preset action, and determining that the action difference degree falls within a preset range, wherein
the target photo is the first photo, and the target preset action is the first preset action; or the target photo is the second photo, and the target preset action is the second preset action.

9. The method according to any one of claims 1 to 8, wherein the method further comprises:
displaying a body shape of the to-be-measured person.

10. The method according to any one of claims 1 to 9, wherein the second body parameter is measured by the first electronic device by controlling the at least eight electrodes to generate at least two electrical signals with different frequencies.

11. The method according to any one of claims 1 to 10, wherein the method is performed by the first electronic device.

12. The method according to any one of claims 1 to 10, wherein the method is performed by a second electronic device, and the second electronic device is communicatively connected to the first electronic device; and
the determining a first body parameter and a second body parameter of a to-be-measured person specifically comprises:
receiving, by the second electronic device, the first body parameter and the second body parameter that are sent by the first electronic device.

13. An electronic device, comprising:

at least one memory, configured to store a program; and
at least one processor, configured to execute the program stored in the memory, wherein when the program stored in the memory is executed, the processor is configured to perform the method according to any one of claims 1 to 12.

**Patentansprüche**

1. Computerimplementiertes Verfahren zum Messen physiologischer Parameter, wobei das Verfahren Folgendes umfasst:

Bestimmen (S101) eines ersten Körperparameters und eines zweiten Körperparameters einer zu messenden Person, wobei der erste Körperparameter ein Körpergewicht ist und der zweite Körperparameter eine Körperimpedanz oder Rohdaten sind, die zum Berechnen einer Körperimpedanz erforderlich sind, und wobei der erste Körperparameter und der zweite Körperparameter durch eine erste elektronische Vorrichtung gemessen werden und der zweite Körperparameter basierend auf mindestens acht Elektroden der ersten elektronischen Vorrichtung gemessen wird;
Bestimmen (S102) eines dritten Körperparameters als Reaktion auf eine Eingabe der zu messenden Person, wobei der dritte Körperparameter eine Körpergröße ist;
Bestimmen (S103) eines vierten Körperparameters basierend auf dem ersten Körperparameter und dem dritten Körperparameter, wobei der vierte Körperparameter ein Body-Mass-Index, BMI, ist;
Bestimmen (S104) einer ersten Körperform der zu messenden Person basierend auf dem zweiten Körperparameter;
Bestimmen (S105) eines ersten physiologischen Parameters basierend auf mindestens der ersten Körperform und dem vierten Körperparameter, wobei der erste physiologische Parameter einen Leberfettwert beinhaltet; und
Anzeigen (S106) des ersten physiologischen Parameters.

2. Verfahren nach Anspruch 1, wobei das Bestimmen einer ersten Körperform der zu messenden Person basierend auf dem zweiten Körperparameter insbesondere Folgendes umfasst:

Bestimmen eines fünften Körperparameters basierend auf dem zweiten Körperparameter, wobei der fünfte Körperparameter ein Taille-Hüft-Verhältnis ist;
wenn der fünfte Körperparameter zu einem ersten Intervall gehört, Bestimmen der ersten Körperform der zu messenden Person basierend auf dem vierten Körperparameter; und
wenn der fünfte Körperparameter zu einem zweiten Intervall gehört, Bestimmen der ersten Körperform der zu messenden Person basierend auf einem sechsten Körperparameter, wobei der sechste Körperparameter basierend auf dem zweiten Körperparameter erlangt wird und wobei der sechste Körperparameter eine oder mehrere der Folgenden beinhaltet: Muskelmasse, Fettmasse von Armen der zu messenden Person.

3. Verfahren nach Anspruch 1 oder 2, wobei das Bestimmen eines ersten physiologischen Parameters basierend auf mindestens der ersten Körperform und dem vierten Körperparameter insbesondere Folgendes umfasst:

Abfragen einer vorbestimmten Entsprechung zwischen einer Körperform und einem Erkennungsmodell basierend auf der ersten Körperform, um ein Erkennungsmodell zu bestimmen, das der ersten Körperform entspricht, wobei unterschiedliche Körperformen unterschiedlichen Erkennungsmodellen in der Entsprechung entsprechen; und
Eingeben von mindestens dem vierten Körperparameter in das Erkennungsmodell, das der ersten Körperform entspricht, um den ersten physiologischen Parameter zu bestimmen.

4. Verfahren nach Anspruch 1, wobei das Verfahren nach dem Anzeigen des ersten physiologischen Parameters ferner Folgendes umfasst:

Bestimmen eines ersten Fotos und eines zweiten Fotos der zu messenden Person als Reaktion auf einen ersten Bedienvorgang der zu messenden Person, wobei das erste Foto aufgenommen wird, wenn die zu messende Person eine erste voreingestellte Aktion ausführt, und das zweite Foto aufgenommen wird, wenn die zu messende Person eine zweite voreingestellte Aktion ausführt;
Bestimmen eines achten Körperparameters der zu messenden Person basierend auf dem ersten Foto und dem zweiten Foto, wobei der achte Körperparameter ein Taillenumfang ist;
Neubestimmen des ersten physiologischen Parameters basierend auf mindestens der ersten Körperform, dem vierten Körperparameter und dem achten Körperparameter; und
Anzeigen des ersten physiologischen Parameters.

5. Verfahren nach Anspruch 4, wobei das Neubestimmen des ersten physiologischen Parameters basierend auf mindestens der ersten Körperform, dem vierten Körperparameter und dem achten Körperparameter insbesondere Folgendes umfasst:

Abfragen einer vorbestimmten Entsprechung zwischen einer Körperform und einem Erkennungsmodell basierend auf der ersten Körperform, um ein Erkennungsmodell, das der ersten Körperform entspricht, zu bestimmen, wobei unterschiedliche Körperformen unterschiedlichen Erkennungsmodellen in der zweiten Entsprechung entsprechen; und
Eingeben von mindestens dem vierten Körperparameter und dem achten Körperparameter in das Erkennungsmodell, das der ersten Körperform entspricht, um den ersten physiologischen Parameter neu zu bestimmen.

6. Verfahren nach Anspruch 5, wobei das Verfahren vor dem Eingeben von mindestens dem vierten Körperparameter und dem achten Körperparameter in das Erkennungsmodell, das der ersten Körperform entspricht, ferner Folgendes umfasst:

Bestimmen eines neunten Körperparameters der zu messenden Person basierend auf dem ersten Foto und dem zweiten Foto, wobei der neunte Körperparameter ein Hüftumfang ist; und
Bestimmen einer zweiten Körperform der zu messenden Person basierend auf dem achten Körperparameter und dem neunten Körperparameter.

7. Verfahren nach Anspruch 6, wobei das Verfahren ferner Folgendes umfasst:

wenn die zweite Körperform nicht mit der ersten Körperform übereinstimmt,
Abfragen der Entsprechung basierend auf der zweiten Körperform, um ein Erkennungsmodell, das der zweiten Körperform entspricht, zu bestimmen, und Auswählen des Erkennungsmodells, das der zweiten Körperform entspricht, um den ersten physiologischen Parameter neu zu bestimmen.

8. Verfahren nach einem der Ansprüche 4 bis 7, wobei das Bestimmen eines ersten Fotos und eines zweiten Fotos der zu messenden Person insbesondere Folgendes umfasst:

Bestimmen eines Aktionsunterschiedsgrads zwischen einer Aktion der zu messenden Person in einem aktuell erlangten Zielfoto und einer voreingestellten Zielaktion und Bestimmen, dass der Aktionsunterschiedsgrad innerhalb eines voreingestellten Bereichs liegt, wobei

das Zielfoto das erste Foto ist und die voreingestellte Zielaktion die erste voreingestellte Aktion ist; oder das Zielfoto das zweite Foto ist und die voreingestellte Zielaktion die zweite voreingestellte Aktion ist.

9. Verfahren nach einem der Ansprüche 1 bis 8, wobei das Verfahren ferner Folgendes umfasst: Anzeigen einer Körperform der zu messenden Person.

10. Verfahren nach einem der Ansprüche 1 bis 9, wobei der zweite Körperparameter durch die erste elektronische Vorrichtung gemessen wird, indem die mindestens acht Elektroden gesteuert werden, um mindestens zwei elektrische Signale mit unterschiedlichen Frequenzen zu erzeugen.

11. Verfahren nach einem der Ansprüche 1 bis 10, wobei das Verfahren durch die erste elektronische Vorrichtung durchgeführt wird.

12. Verfahren nach einem der Ansprüche 1 bis 10, wobei das Verfahren durch eine zweite elektronische Vorrichtung durchgeführt wird und die zweite elektronische Vorrichtung kommunikativ mit der ersten elektronischen Vorrichtung verbunden ist; und

das Bestimmen eines ersten Körperparameters und eines zweiten Körperparameters einer zu messenden Person insbesondere Folgendes umfasst:
Empfangen, durch die zweite elektronische Vorrichtung, des ersten Körperparameters und des zweiten Körperparameters, die durch die erste elektronische Vorrichtung gesendet werden.

13. Elektronische Vorrichtung, umfassend:

mindestens einen Speicher, der dazu konfiguriert ist, ein Programm zu speichern; und
mindestens einen Prozessor, der dazu konfiguriert ist, das in dem Speicher gespeicherte Programm auszuführen, wobei, wenn das in dem Speicher gespeicherte Programm ausgeführt wird, der Prozessor dazu konfiguriert ist, das Verfahren nach einem der Ansprüche 1 bis 12 durchzuführen.

## Revendications

1. Procédé mis en œuvre par ordinateur pour mesurer des paramètres physiologiques, dans lequel le procédé comprend :

la détermination (S101) d'un premier paramètre corporel et d'un deuxième paramètre corporel d'une personne à mesurer, dans lequel le premier paramètre corporel est un poids corporel et le deuxième paramètre corporel est une impédance corporelle ou des données brutes requises pour calculer une impédance corporelle, et dans lequel le premier paramètre corporel et le deuxième paramètre corporel sont mesurés par un premier dispositif électronique, et le deuxième paramètre corporel est mesuré sur la base d'au moins huit électrodes du premier dispositif électronique ;
la détermination (S102) d'un troisième paramètre corporel en réponse à une entrée de la personne à mesurer, dans lequel le troisième paramètre corporel est une taille corporelle ;
la détermination (S103) d'un quatrième paramètre corporel sur la base du premier paramètre corporel et du troisième paramètre corporel, dans lequel le quatrième paramètre corporel est un indice de masse corporelle, IMC ;
la détermination (S104) d'une première forme corporelle de la personne à mesurer sur la base du deuxième paramètre corporel ;
la détermination (S105) d'un premier paramètre physiologique sur la base d'au moins la première forme corporelle et du quatrième paramètre corporel, dans lequel le premier paramètre physiologique comporte un niveau de graisse hépatique ; et
l'affichage (S106) du premier paramètre physiologique.

2. Procédé selon la revendication 1, dans lequel la détermination d'une première forme corporelle de la personne à mesurer, sur la base du deuxième paramètre corporel, comprend spécifiquement :

la détermination d'un cinquième paramètre corporel sur la base du deuxième paramètre corporel, dans lequel le cinquième paramètre corporel est un rapport taille-hanches ;
si le cinquième paramètre corporel appartient à un premier intervalle, la détermination de la première forme corporelle de la personne à mesurer sur la base du quatrième paramètre corporel ; et
si le cinquième paramètre corporel appartient à un second intervalle, la détermination de la première forme corporelle de la personne à mesurer sur la base d'un sixième paramètre corporel, dans lequel le sixième paramètre corporel est obtenu sur la base du deuxième paramètre corporel, et dans lequel le sixième paramètre corporel comporte l'une ou plusieurs de la masse musculaire, de la masse grasse des bras de la personne à mesurer.

3. Procédé selon la revendication 1 ou 2, dans lequel la détermination d'un premier paramètre physiologique, basée au moins sur la première forme corporelle et le quatrième paramètre corporel, comprend spécifiquement :

l'interrogation d'une correspondance prédéterminée entre une forme corporelle et un modèle de détection basé sur la première forme corporelle, pour déterminer un modèle de détection correspondant à la première forme corporelle, dans lequel différentes formes corporelles correspondent à différents modèles de détection dans la correspondance ; et
l'entrée au moins du quatrième paramètre corporel dans le modèle de détection correspondant à la première forme corporelle, pour déterminer le premier paramètre physiologique.

4. Procédé selon la revendication 1, dans lequel, après l'affichage du premier paramètre physiologique, le procédé comprend également :

la détermination d'une première photo et d'une seconde photo de la personne à mesurer en réponse à une première opération de la personne à mesurer, dans lequel la première photo est prise lorsque la personne à mesurer réalise une première action prédéfinie, et la seconde photo est prise lorsque la personne à mesurer réalise une seconde action prédéfinie ;
la détermination d'un huitième paramètre corporel de la personne à mesurer sur la base de la première photo et de la seconde photo, dans lequel le huitième paramètre corporel est un tour de taille ;
la redétermination du premier paramètre physiologique sur la base au moins de la première forme corporelle, du quatrième paramètre corporel et du huitième paramètre corporel; et
l'affichage du premier paramètre physiologique.

5. Procédé selon la revendication 4, dans lequel la redétermination du premier paramètre physiologique, sur la base au moins de la première forme corporelle, du quatrième paramètre corporel et du huitième paramètre corporel, comprend spécifiquement :

l'interrogation d'une correspondance prédéterminée entre une forme corporelle et un modèle de détection basé sur la première forme corporelle, pour déterminer un modèle de détection correspondant à la première forme corporelle, dans lequel différentes formes corporelles correspondent à différents modèles de détection dans la seconde correspondance ; et
l'entrée au moins du quatrième paramètre corporel et du huitième paramètre corporel dans le modèle de détection correspondant à la première forme corporelle, pour redéterminer le premier paramètre physiologique.

6. Procédé selon la revendication 5, dans lequel, avant l'entrée au moins du quatrième paramètre corporel et du huitième paramètre corporel dans le modèle de détection correspondant à la première forme corporelle, le procédé comprend également :

la détermination d'un neuvième paramètre corporel de la personne à mesurer sur la base de la première photo et de la seconde photo, dans lequel le neuvième paramètre corporel est un tour de hanches ; et
la détermination d'une seconde forme corporelle de la personne à mesurer sur la base du huitième paramètre corporel et du neuvième paramètre corporel.

7. Procédé selon la revendication 6, dans lequel le procédé comprend également :

si la seconde forme corporelle est incompatible avec la première forme corporelle,
l'interrogation de la correspondance sur la base de la seconde forme corporelle, pour déterminer un modèle de détection correspondant à la seconde forme corporelle, et la sélection du modèle de détection correspondant à la seconde forme corporelle pour redéterminer le premier paramètre physiologique.

8. Procédé selon l'une quelconque des revendications 4 à 7, dans lequel la détermination d'une première et d'une seconde photo de la personne à mesurer comprend spécifiquement :

la détermination d'un degré de différence d'action entre une action de la personne à mesurer dans une photo cible actuellement obtenue et une action cible prédéfinie, et la détermination selon laquelle le degré de différence d'action se situe dans une plage prédéfinie, dans lequel
la photo cible est la première photo et l'action prédéfinie cible est la première action prédéfinie ; ou la photo cible est la seconde photo et l'action prédéfinie cible est la seconde action prédéfinie.

9. Procédé selon l'une quelconque des revendications 1 à 8, dans lequel le procédé comprend également l'affichage d'une forme corporelle de la personne à mesurer.

10. Procédé selon l'une quelconque des revendications 1 à 9, dans lequel le deuxième paramètre corporel est mesuré par le premier dispositif électronique en commandant les au moins huit électrodes pour générer au moins deux signaux électriques avec des fréquences différentes.

11. Procédé selon l'une quelconque des revendications 1 à 10, dans lequel le procédé est réalisé par le premier dispositif électronique.

12. Procédé selon l'une quelconque des revendications 1 à 10, dans lequel le procédé est réalisé par un second dispositif électronique, et le second dispositif électronique est connecté en communication au premier dispositif électronique ; et
la détermination d'un premier paramètre corporel et d'un deuxième paramètre corporel d'une personne à mesurer comprend spécifiquement :
la réception, par le second dispositif électronique, du premier paramètre corporel et du deuxième paramètre corporel qui sont envoyés par le premier dispositif électronique.

13. Dispositif électronique, comprenant :

au moins une mémoire, configurée pour stocker un programme ; et
au moins un processeur, configuré pour exécuter le programme stocké dans la mémoire, dans lequel lorsque le programme stocké dans la mémoire est exécuté, le processeur est configuré pour réaliser le procédé selon l'une quelconque des revendications 1 à 12.

11

12

FIG. 1a

11

12

13

FIG. 1b

221    22    221

221    221

200

23

211    211

211    211

21

FIG. 2

Electronic device 300

Camera 305

Processor 301

Memory 302

Display 304

Communication module 303

FIG. 3

A

22

211

211

200

21

FIG. 4

FIG. 5

FIG. 6

Name: A

Weight
79 Kg

Height
178 cm

Liver fat level
7.8
Medium or high risk

Exercise recommendation:
xx

300

Next Page

61

FIG. 7

The photographing
function needs to be
enabled

OK

Cancel

72

71

FIG. 8

Name: A

Weight
79 Kg

Height
178 cm

Liver fat level
7.8
Medium or high risk

Exercise recommendation:
xx

Photo
measurement       OK

300

62

FIG. 9a

Name: A

Weight
79 Kg

Height
178 cm

Liver fat level
7.8
Medium or high risk

Exercise recommendation:
xx

Photo
measurement       Yes   No

300

63

FIG. 9b

FIG. 10a

FIG. 10b

FIG. 10c

FIG. 10d

FIG. 10e

FIG. 10f

The photographing function
needs to be enabled

92

OK

Cancel

91

FIG. 11a

Photographing recommendation:

xx

93

OK

Cancel

FIG. 11b

901

FIG. 11c

901

FIG. 11d

Measuring
55%

FIG. 11e

Liver fat level: xx

Body shape: xx

Exercise recommendation: xx

FIG. 11f

Bust circumference: xx

Waist circumference: xx

Hip circumference: xx

Waist-to-hip ratio: xx

FIG. 11g

Determine a first body parameter and a second body parameter of a to-be-measured person    S101

↓

Determine a third body parameter in response to an input of the to-be-measured person    S102

↓

Determine a fourth body parameter based on the first body parameter and the third body parameter    S103

↓

Determine a first body shape of the to-be-measured person based on the second body parameter    S104

↓

Determine a first physiological parameter based on at least the first body shape and the fourth body parameter    S105

↓

Display the first physiological parameter    S106

FIG. 12

```
┌─────────────────────────────────────────────────────────┐  S201
│ Determine a first photo and a second photo of a to-be-   │
│ measured person in response to a first operation of the  │
│ to-be-measured person                                    │
└─────────────────────────────────────────────────────────┘
                            │
                            ▼
┌─────────────────────────────────────────────────────────┐  S202
│ Determine an eighth body parameter of the to-be-measured │
│ person based on the first photo and the second photo     │
└─────────────────────────────────────────────────────────┘
                            │
                            ▼
┌─────────────────────────────────────────────────────────┐  S203
│ Redetermine a first physiological parameter based on at  │
│ least a first body shape, a fourth body parameter, and   │
│ the eighth body parameter                                │
└─────────────────────────────────────────────────────────┘
                            │
                            ▼
┌─────────────────────────────────────────────────────────┐  S204
│ Display the first physiological parameter                │
└─────────────────────────────────────────────────────────┘
```

FIG. 13

```
┌─────────────────────────────────────────────────────────┐
│          System for detecting physiological parameters   │
│                                                          │
│   ┌──────────────────┐           ┌──────────────────┐    │
│   │  First electronic │   /\/     │ Second electronic│    │
│   │   device 1401     │           │   device 1402    │    │
│   └──────────────────┘           └──────────────────┘    │
│                                                          │
└─────────────────────────────────────────────────────────┘
```

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110531351
- JP 4723078 B
- US 2002123695 A1
- US 2009018464 A1
- US 2010106045 A1

**Non-patent literature cited in the description**

- **ORKIN SARAH et al.** Body composition measured by bioelectrical impedance analysis is a viable alternative to magnetic resonance imaging in children with nonalcoholic fatty liver disease. *JPEN - Journal of Parenteral and Enteral Nutrition*, 30 April 2021, vol. 46 (2), 378-384